# EUROPÄISCHE PATENTSCHRIFT

(11) **EP 3 284 510 B1**
(45) Veröffentlichungstag und Bekanntmachung des Hinweises auf die Patenterteilung: **11.11.2020**
(21) Anmeldenummer: 16184299.2
(22) Anmeldetag: 16.08.2016
(51) Int. Cl.: A61N 1/08, A61N 1/37

(54) **ELEKTRODENLEITUNG, IMPLANTAT UND VERFAHREN ZUR IDENTIFIKATION EINER ELEKTRODENLEITUNG**
ELECTRODE LEAD, IMPLANT AND METHOD FOR THE IDENTIFICATION OF AN ELECTRODE LEAD
LIGNE D'ELECTRODES, IMPLANT ET PROCEDE D'IDENTIFICATION D'UNE LIGNE D'ELECTRODES

(43) Veröffentlichungstag der Anmeldung: 21.02.2018
(73) Patentinhaber: BIOTRONIK SE & Co. KG, 12359 Berlin (DE)
(72) Erfinder: Weiss, Ingo, 12435 Berlin (DE)
(74) Vertreter: Randoll, Sören

(56) Entgegenhaltungen:
- US-A- 5 755 742
- US-A1- 2003 018 369
- US-A1- 2004 073 265
- US-A1- 2011 112 609
- US-A1- 2014 343 633

## Beschreibung

Die Erfindung betrifft eine Elektrodenleitung, ein Verfahren zur Identifikation einer Elektrodenleitung sowie ein Implantat mit einer Buchse zur Verbindung mit einer Elektrodenleitung.

Implantate wie Herzschrittmacher (Pacemaker), Defibrillatoren oder neurologische Geräte wie Neurostimulatoren, z. B. Hirnschrittmacher für tiefe Hirnstimulation (Deep Brain Stimulation), Rückenmarkstimulationsgeräte, TENS (Transcutaneous Electrical Nerve Stimulator), Geräte für muskuläre Stimulationstherapie oder Diagnosegeräte, welche die chemischen Eigenschaften des Blutes des Patienten, anderer Körperteile oder andere Körpereigenschaften und -parameter untersuchen, oder vergleichbare implantierbare Vorrichtungen verwenden häufig Elektrodenleitungen, die in den Körper des Patienten geführt werden und dort zumindest über den Zeitraum der Behandlung oder Messung verbleiben. Die Elektrodenleitungen sind mit dem Implantat elektrisch leitend verbunden.

Die Implantate umfassen üblicherweise ein körperverträgliches Gehäuse mit einer dazugehörigen elektronischen Schaltung und einer Energieversorgung, z. B. einer Batterie. Das Gehäuse besitzt mindestens eine Buchse, an der eine oder mehrere Elektrodenleitungen angeschlossen werden können, beispielsweise mittels eines Steckers. Eine Elektrodenleitung dient der Übertragung der elektrischen Energie bzw. von Daten vom Gehäuse zu dem behandelten oder untersuchten Körperteil und umgekehrt.

Im Rahmen der vorliegenden Erfindung wird unter dem Begriff "Elektrodenleitung" eine Leitung mit einem oder mehreren elektrischen Leitern zusammen mit seiner umhüllenden Isolation, die häufig als Isolationsschlauch ausgebildet ist, sowie alle weiteren funktionellen Elemente, die mit der Leitung fest verbunden sind, verstanden. Die Elektrodenleitung umfasst wenigstens einen Elektrodenpol am distalen Ende, durch den elektrische Signale an das Gewebe übertragen werden oder mit dem elektrische Signale vom Gewebe abgeleitet werden können. Die Elektrodenleitung kann eine sogenannte Elektrodenspitze als Elektrodenpol umfassen, mittels der die elektrische Energie in das zu behandelnde Gewebe eingeleitet wird. Häufig ist eine Elektrodenspitze mit Ankerelementen oder Haltestrukturen versehen, mit denen die Konstanz der räumlichen Lage der Übergangsstelle der elektrischen Energie in das zu behandelnde Gewebe sichergestellt wird. Die Elektrodenspitze kann als Ableit-, Stimulations- oder Messelektrode ausgebildet sein. Auch kann die Elektrodenleitung einen oder mehrere ringförmige Elektrodenpole aufweisen, mittels derer die elektrische Energie in das zu behandelnde Gewebe eingeleitet wird. Die ringförmigen Elektrodenpole können als Ableit-, Stimulations- oder Messelektroden ausgebildet sein. Weiter ist am proximalen Ende der Elektrodenleitung häufig ein Stecker ausgebildet, der zum elektrisch leitenden Anschluss der Elektrodenleitung an ein elektronisches Gerät, hier insbesondere an ein Implantat, dient.

An moderne Implantate, beispielsweise einen Mehrkammer-Herzschrittmacher oder einen implantierbaren Kardioverter-Defibrillator (ICD), werden häufig mehrere Elektrodenleitungen angeschlossen. Hierbei besteht das Bestreben, die Elektrodenleitungen und ihre Anschlüsse möglichst dünn bzw. klein zu gestalten. Dies erschwert die gut sichtbare Markierung der Stecker und Anschlüsse sowie die Unterscheidung der Elektrodenleitungen. Darüber hinaus steigt mit zunehmender Anzahl von Elektrodenleitungen die Gefahr, dass einzelne Elektrodenleitungen verwechselt und/oder falsch angeschlossen werden. Deshalb ist es wünschenswert, wenn ein Implantat erkennt, welche Elektrodenleitungen angeschlossen sind, so dass es diese adäquat ansteuern kann. Dazu müssen die Elektrodenleitungen und/oder ihre Eigenschaften für das Implantat identifizierbar sein.

Dokument US 2004/0073265 A1 beschreibt eine Vorrichtung, die eine Möglichkeit bietet, falsch verbundene Koronarleitungen und/oder falsche Verbindungen zu Herzrhythmusmanagement-Vorrichtungen zu erkennen. Dazu erzeugt eine Spannungseinbringungsvorrichtung eines Schrittmachers einen Spannungspuls zwischen einer Elektrode, die mit einer Leitung mit dem Schrittmacher verbunden ist, und einer Kopf- oder Gehäuseelektrode des Schrittmachers. Die Gehäuseelektrode sendet ein Verbindungssignal. Die Elektrode wird genutzt, um ein korrespondierendes Verbindungssignal unter Nutzung der Leitung zu messen. Ein Messmodul der Vorrichtung misst weiter eine oder mehrere Eigenschaften des korrespondierenden Verbindungssignals wie seine Stromstärke, Spannung, Impedanz und/oder sein Zeitverzug (nach Aussendung des Spannungspulses). Die Signaleigenschaften können von einer oder mehreren Leitungen und/oder vom übermittelnden Gewebe und Flüssigkeiten (beispielsweise einem Herz inklusive einer oder mehrere seiner Kammern), die dazwischen liegen, beeinflusst werden. Ein Vergleichsmodul des Schrittmachers kann daraufhin feststellen, ob die Leitung ordnungsgemäß zu einem Kontakt des Schrittmachers ist, wobei eine oder mehrere Eigenschaften des korrespondierenden Verbindungssignals mit geeigneten vorgewählten Wertebereichen verglichen werden. Beispielsweise kann eine gemessene Impedanz mit einem erwarteten Impedanzbereich verglichen werden. Die in der Druckschrift beschriebene Vorrichtung identifiziert damit nicht selektiv die Leitung, sondern testet vielmehr, ob das korrespondierende Vergleichssignal, das über eine Leitung nach Anregung des Körpers durch einen Spannungspuls einer Gehäuseelektrode des Schrittmachers empfangen wird, Eigenschaften innerhalb eines vorgegebenen Wertebereichs aufweist. Die Eigenschaften des korrespondierenden Vergleichssignals werden auch durch das angeregte Körpergewebe zwischen der Gehäuseelektrode des Schrittmachers und der empfangenden Elektrode bestimmt. Nur sehr grobe Abweichungen, wie sie durch eine nicht verbundene oder vollkommen falsche Art von Leitung auftreten, können sicher auf die Leitung zurückgeführt werden, geringere Abweichungen können körperbedingt sein. Die oben genannte Vorrichtung kann damit die sichere und gezielte Erkennung und Unterscheidung von Elektrodenleitungen mit ähnlichen Eigenschaften nicht gewährleisten.

Eine ähnliche Vorrichtung wird auch in Dokument US 2006/0212083 A1 offenbart. Auch in dieser Druckschrift wird betont, dass die Signaleigenschaften von den Leitungen und/oder von dem/der dazwischen liegenden übermittelnden Gewebe und Flüssigkeit beeinflusst werden.

Dokument US 2011/0112609 A1, das als nächstliegender Stand der Technik angesehen wird, beschreibt ein System zur Rückenmarksstimulation mit mindestens einer implantierbaren Stimulationsleitung. Es umfasst insbesondere ein ärztliches Programmiergerät und einen implantierbaren Pulsgenerator, der mit einer oder mehreren implantierbaren Stimulationsleitungen verbunden ist, die je eine Vielzahl von Elektroden tragen. Die Stimulationsleitung weist ein oder zwei Leitungskörper auf. Die Elektroden passen genau in den Epiduralraum der Wirbelsäule. Da das dortige Gewebe leitend ist, können elektrische Messungen zwischen den Elektroden durchgeführt werden. Ein Kontrollschaltkreis des implantierbaren Pulsgenerators erfasst solche elektrischen Messungen, so dass das ärztliche Programmiergerät automatisch die einzelnen Leitungskörper identifizieren kann, die mit dem implantierbaren Pulsgenerator verbunden sind. Die elektrischen Messungen des Kontrollschaltkreises zur Identifikation der verbundenen Leitungskörper sind Feldpotentiale. Der Kontrollschaltkreis kann auch die Impedanz an jeder Elektrode messen, um die Kopplungseffizienz zwischen der jeweiligen Elektrode und dem Gewebe zu bestimmen und um die Fehlerkennung bezüglich der Verbindung zwischen der Elektrode und dem analogen Ausgabeschaltkreis des implantierbaren Pulsgenerators zu bestimmen. Es ist erforderlich, dass die implantierbare Stimulationsleitung zur elektrischen Messung und der hieraus folgenden Identifikation der Leitungskörper bereits implantiert sein muss. Die Messung wird erst durch das Körpergewebe ermöglicht und von ihm beeinflusst. Wünschenswert wäre jedoch eine Identifikation lediglich durch die implantierbare Stimulationsleitung und auch im nicht implantierten Zustand. Zudem ist bei dem bekannten System von Nachteil, dass die Identifikation nicht durch den implantierbaren Pulsgenerator selbst, sondern durch ein zusätzliches ärztliches Programmiergerät erfolgt.

Dokument US 2012/0123496 A1 beschreibt die Erkennung der Verbindung und die Identifikation des Typs einer implantierten Leitung für eine implantierte medizinische Vorrichtung. Die Vorrichtung weist einen Prozessor auf, der die Verbindung sowie den Leitungstyp der Leitung bestimmen kann. Hierbei beginnt die Benutzung der implantierbaren Vorrichtung, der Leitung und der zugehörigen Methode erst, wenn die Leitung bereits implantiert und mit der implantierbaren Vorrichtung verbunden ist. Zunächst prüft ein Signalmessmodul die Verbindung der Leitungen, indem es Werte elektrischer Parameter während eines Signals zwischen zumindest zwei Elektroden prüft, insbesondere der Impedanz. Eine oder mehrere Leitungen können darin integrierte, aktive Elektronik aufweisen, die einen oder mehrere darin integrierte modulare Schaltkreise beinhaltet, je nachdem, ob die Leitung unipolar oder multipolar ist. Jeder der modularen Schaltkreise ist in der Lage, eine Vielzahl von Elektroden der Leitung zu steuern, und schließt eine Schaltungsanordnung ein, die elektrisch mit einer oder mehreren Elektroden der Leitung verbunden ist. Als solcher wirkt jeder der modularen Schaltkreise einer Leitung als Schnittstelle zwischen der implantierten medizinischen Vorrichtung und den Elektroden, mit denen der modulare Schaltkreis verbunden ist. Für die Messung der Impedanz steuert der Prozessor der Vorrichtung den modularen Schaltkreis, sodass dieser einen Spannungspuls zwischen einer ersten und einer zweiten Elektrode liefert. Das Signalmessmodul misst den resultierenden Strom und der Prozessor leitet den Impedanzwert ab. In einem weiteren Schritt sendet der Prozessor ein Abfragesignal entlang eines ersten Leiters der Leitung, um eine Antwort von den modularen Schaltkreisen über eine zweite Leitung zu erhalten. Eine solche Antwort von jedem modularen Schaltkreis liefert dem Prozessor Informationen zu dem modularen Schaltkreis und den Elektroden, die er steuert. In einem weiteren Konfigurationsschritt sendet der Prozessor ein Signal über die erste Leitung. Der Konfigurationsschritt schließt ein, dass die aktive Konfiguration der modularen Schaltkreise programmiert wird. Bezüglich Leitungsausführungen und darin verwendeter aktiver Elektronik bzw. modularer Schaltkreise wird auf das Dokument US 7,713,194 verwiesen. Gemäß dieser Druckschrift ist der modulare Schaltkreis derart ausgestaltet, dass er durch einen Bus gesteuert wird. Die Druckschrift US 2012/0123496 A1 beschreibt demnach, dass die zusätzliche Schnittstellenelektronik modularer Schaltkreise erkannt und somit die Elektrodenleitung bestimmt werden kann. Allerdings muss die Elektrodenleitung dafür bereits implantiert sein. Nur dann ist auch die vorher erforderliche Messung der Impedanz zwischen zwei Elektroden möglich. Bei der bekannten Vorrichtung ist weiter nachteilig, dass komplexe modulare Schaltkreise mit aktiver Elektronik zur Steuerung der Elektroden implementiert und programmiert werden müssen, was auch im Hinblick auf die Kosten aufwändig ist.

Ein Verfahren und eine Vorrichtung zur automatischen Erkennung von implantierbaren medizinischen Leitungen und deren Konfiguration sind in dem Dokument US 2003/0018369 A1 dargestellt. Dafür ist ein erster Kommunikationsschaltkreis, der Daten wie Modell- und Seriennummer, technische Information und Kalibrierungsdaten speichert, mit der Leitung verbunden oder in ihr integriert. Dieser weist einen Empfänger und einen Sender auf, um Datensignale von einer externen Quelle zu empfangen. So kann er bei der Herstellung mit Identifikationsdaten, Kalibrierungsdaten und anderen Daten programmiert werden. Der erste Kommunikationsschaltkreis ist als passiver Transponder ausgeführt und weist neben dem Receiver und Transmitter weiter einen Energiekoppler zur Energieversorgung und einen Steuerschaltkreis auf, welcher mit einem nicht-flüchtigen Speicher verbunden ist. Der Steuerschaltkreis liefert die Informationen zum Transmitter/Receiver des Transponders, der die Daten via RF oder anderer Kommunikation übermittelt. Während der Implantation der Leitung oder danach können die Informationen zu einem zweiten Kommunikationsschaltkreis außerhalb der Leitung transferiert werden. Die transferierten Daten können zur Identifikation der Leitung genutzt werden, in eine Patientenakte aufgenommen und in einen Zentralspeicher für die Nutzung durch Gesundheitsdienstleister transferiert werden. Anhand des Transponders kann die Leitung automatisch erkannt werden und die im Speicher abgelegten Daten können direkt transferiert und weitergegeben werden. Der Transponder benötigt neben einem Transmitter und Receiver allerdings eine separate Energieversorgung, eine Steuereinheit und einen programmierbaren, digitalen Speicher. Dadurch ist der Gesamtaufbau der Leitung vergleichsweise aufwendig und teuer.

Auch Dokument US 2014/0343633 A1 stellt eine elektrisch identifizierbare Elektrodenleitung mit einem Identifikationsmodul, welches zumindest einen Filter, einen Stromkonverter, eine Kommunikationsschaltung, einen Lastschalter und eine Speichereinheit wie ein EPROM zur Speicherung eines Identifikationscodes aufweist. Vor der Einbringung des Implantats wird jede Leitung implantiert und mit dem implantierbaren Pulsgenerator (oder einem externen Pulsgenerator) verbunden, welcher dann selbstidentifizierende Daten vom Identifikationsmodul ausliest und diese Information an eine externe Vorrichtung wie den Arztprogrammierer übermitteln kann. Dafür kann das Identifikationsmodul bis zu 32 Byte Daten speichern. Dieses Verfahren wird für jede Leitung, die implantiert wird, wiederholt. Das Identifikationsmodul nutzt zwei vorhandene Kontakte der Leitung zur Verbindung mit dem implantierbaren Pulsgenerator. Wenn das Identifikationsmodul gerade nicht kommuniziert, ist es für einen Stimulationskreis des implantierbaren Pulsgenerators unsichtbar, da seine Impedanz zwischen den Kontakten größer als 1 MΩ ist. Das Identifikationsmodul muss gegen Ströme, die in Gewebe durch externe Quellen wie Defibrillatorpulse, Elektrokauterisierung oder Magnetresonanztomographie auftreten, geschützt werden. Wie im zuvor genannten Dokument wird auch für diese bekannte Elektrodenleitung ein digitaler Speicher benötigt und der Aufbau des Identifikationsmoduls ist ähnlich komplex. Weiter von Nachteil sind die Anforderungen an die Impedanz des Identifikationsmoduls und der notwendige Schutz gegen im Gewebe auftretende Ströme.

Die Aufgabe der vorliegenden Erfindung besteht deshalb darin, eine Elektrodenleitung zu schaffen, die zuverlässig identifiziert werden kann, aber zugleich einfach aufgebaut ist und kostengünstig hergestellt werden kann.

Die obige Aufgabe wird gelöst durch eine Elektrodenleitung mit den Merkmalen des Anspruchs 1.

Die erfindungsgemäße Elektrodenleitung zur Verbindung mit einem Implantat mittels eines Steckers, weist einen analogen elektrischen ID-Schaltkreis auf, der in der Elektrodenleitung steckerseitig angeordnet und mit einer steckerseitig angeordneten Koppeleinrichtung verbunden ist, wobei der ID-Schaltkreis über die Koppeleinrichtung anregbar und derart ausgestaltet ist, dass während oder nach Anregung eine charakteristische Größe des ID-Schaltkreises über die Koppeleinrichtung und/oder über eine mit dem ID-Schaltkreis verbundene, steckerseitig angeordnete Auskoppeleinrichtung messbar ist und dass auf der Basis einer gemessenen Kennlinie der charakteristischen Größe mindestens eine individuelle Eigenschaft der Elektrodenleitung, beispielsweise die zugehörige Seriennummer, Losnummer, Typ, die Ausführung, Revision oder dergl., bestimmbar ist. Der ID-Schaltkreis ist ferner passiv ausgeführt, d.h. er weist keine aktive Spannungs- oder Stromquelle auf.

Unter einem analogen elektrischen ID-Schaltkreis wird ein analoger Schaltkreis verstanden, der ausschließlich (nur) mindestens ein analoges elektrisches Bauelement aufweist, wobei mehrere solche Bauelemente zu einem Schaltkreis verbunden sind. Ein analoges elektrisches Bauelement ist im Rahmen der vorliegenden Erfindung ein ohmscher Widerstand, ein Kondensator, eine Diode, eine Spule, ein Transistor oder ein Transformator, wobei die Bauelemente sowohl in einer diskreten Schaltung als auch in einer integrierten Schaltung vorliegen können. Den analogen elektrischen ID-Schaltkreis gemäß der vorliegenden Erfindung kennzeichnet außerdem, dass aus einer zeitlich stetigen Anregung eine zeitlich stetige Reaktion des Schaltkreises resultiert.

Die erfindungsgemäße Elektrodenleitung hat den Vorteil, dass sie einfach und kostengünstig realisierbar ist, da passive analoge elektrische Schaltkreise vergleichsweise wenig aufwendig in der Herstellung sind. Weiter kann ein solcher Schaltkreis durch die Dimensionierung der analogen Bauelemente einfach an die Vorgaben zu der individuellen Eigenschaft der Elektrodenleitung angepasst werden.

Die erfindungsgemäße Elektrodenleitung bietet den Vorteil, dass ein mit ihr verbundenes Implantat anhand der mindestens einen individuellen Eigenschaft prüfen kann, ob die angeschlossene Elektrodenleitung für den Anschluss und/oder die geplante Verwendung geeignet ist. Insbesondere ist es möglich, dass ein erfindungsgemäßes Implantat, zum Beispiel ein implantierbarer Herzschrittmacher, Neurostimulator oder Kardioverter-Defibrillator, angeschlossene erfindungsgemäße Elektrodenleitungen identifiziert, sodass der implantierbaren Vorrichtung bekannt ist, mit welcher Elektrodenleitung sie verbunden ist. Damit können Verwechslungen von Elektrodenleitungen mit unterschiedlichen individuellen Eigenschaften erkannt werden.

Die erfindungsgemäße Elektrodenleitung ermöglicht es, den ID-Schaltkreis im Rahmen der Fertigung selbst einzustellen, ohne auf Halbleiterbauelemente Dritter zurückgreifen zu müssen. Insbesondere müssen keine digitalen Speichervorrichtungen in die Elektrodenleitung integriert werden. Dadurch sinken die Herstellungskosten. Außerdem können sehr langlebige und biokompatible Materialien zum Einsatz kommen. Auch können geometrische Randbedingungen der Elektrodenleitungsgestaltung leichter erfüllt werden.

Bei der erfindungsgemäßen Elektrodenleitung kann ferner auf den Einsatz von RFID-Komponenten verzichtet werden. Sie stellt damit eine Alternative zu Identifizierungslösungen unter Verwendung von RFID-Techniken dar.

Ein weiterer Vorteil der Erfindung ist, dass ein abfragendes Implantat keine zusätzliche Technologie zur Messung der charakteristischen Größe des ID-Schaltkreises benötigt. Vielmehr können die vorhandenen Messeinrichtungen durch Modifikation der Ansteuerung zur Messung genutzt werden.

Darüber hinaus ist durch die erfindungsgemäße Elektrodenleitung sichergestellt, dass die mindestens eine individuelle Eigenschaft auf der Basis mindestens einer gemessenen Kennlinie der charakteristischen Größe des ID-Schaltkreises selbst zuverlässig bestimmt werden kann. Die charakteristische Größe ist hierbei eine feste und inhärente Eigenschaft des ID-Schaltkreises selbst, die nach seiner Fertigstellung nicht mehr geändert werden kann.

Vorzugsweise ist der Stecker derart ausgestaltet, dass die Elektrodenleitung mit dem erfindungsgemäßen Implantat, zum Beispiel einem implantierbaren Herzschrittmacher, Neurostimulator oder Kardioverter-Defibrillator, verbunden und wieder von ihm getrennt werden kann.

Ein Vorteil der vorliegenden Erfindung ist, dass im Rahmen des Implantationsprozesses auch außerhalb des Körpers, in den die Implantation erfolgen soll, die mindestens eine individuelle Eigenschaft der Elektrodenleitung identifiziert werden und - falls sie mit einer falschen Buchse des Implantats verbunden ist - wieder abgetrennt und gegebenenfalls an eine andere Buchse angeschlossen werden kann. Die Identifizierung der individuellen Eigenschaft kann auch während oder nach dem Abschluss der Implantation des Implantats und/oder der Elektrodenleitung in den Körper erfolgen. Durch die Identifizierung der Elektrodenleitung wird das Implantat selbst in die Lage versetzt, an einen Port (Buchse) angeschlossene Elektrodenleitungen zu erkennen. Das Implantat erhält hierdurch die Möglichkeit, durch eine auf die Elektrode abgestimmte Ansteuerung der Ports die Elektrodenleitung entsprechend ihrer Bestimmung zu nutzen.

Da der ID-Schaltkreis in der Elektrodenleitung angeordnet ist, ist er in vorteilhafter Weise, wenn die erfindungsgemäße Elektrodenleitung implantiert wurde, vom umgebenden Körpergewebe vollständig abgetrennt.

In einer weiteren bevorzugten Ausführungsform der erfindungsgemäßen Elektrodenleitung ist der ID-Schaltkreis nicht mit einem therapeutischen oder diagnostischen Leiter der Elektrodenleitung elektrisch verbunden, d.h. von einem solchen elektrisch isoliert. Hierbei wird unter einem therapeutischen oder diagnostischen Leiter der Elektrodenleitung die elektrisch leitende Verbindung zwischen Stecker und Elektrodenpol (z. B. Elektrodenspitze oder Ringelektrode) verstanden, welche zur Energie- und/oder Datenübertragung gemäß der herkömmlichen Funktion der Elektrodenleitung zur Messung von Körpersignalen und/oder Übertragung von Stimulationspulsen dient. Dadurch ist der ID-Schaltkreis unabhängig von den für die Therapie oder Diagnostik interessanten Signalen und muss nicht gegen diese geschützt werden. Ferner muss der ID-Schaltkreis auch nicht gegen im Gewebe auftretende Ströme geschützt werden, so dass eine weitere Vereinfachung erreicht wird.

Der analoge ID-Schaltkreis ist über eine Koppeleinrichtung anregbar. Insbesondere kann durch die Anregung Energie in den ID-Schaltkreis eingebracht werden.

In einer bevorzugten Ausführungsform der Erfindung findet die Messung nur über die Koppeleinrichtung statt. Dies bietet den Vorteil, dass keine zusätzliche Auskoppeleinrichtung vorgesehen sein muss. So wird die Komplexität verringert und die Herstellung wird einfacher und kostengünstiger.

Die Messung kann auch nur über die Auskoppeleinrichtung erfolgen. Damit werden für Anregung und Messung zwei unterschiedliche Einrichtungen benutzt. Der Vorteil ist, dass sich Messsignale zur charakteristischen Größe einfacher von der Anregung trennen lassen.

Die Koppeleinrichtung kann als galvanische Koppeleinrichtung oder nichtgalvanische Koppeleinrichtung ausgeführt sein. Eine galvanische Koppeleinrichtung ist besonders einfach herstellbar. Zudem bietet Sie den Vorteil, dass sie die besonders effiziente Übertragung von elektrischer Energie ermöglicht. Eine nichtgalvanische Koppeleinrichtung bietet den Vorteil, dass für sie keine direkte, elektrisch leitende Verbindung zwischen der Elektrodenleitung und dem Implantat zur Anregung und/oder Messung notwendig ist. Stattdessen ist eine drahtlose Anregung und/oder Messung möglich. Insbesondere kann auf elektrisch leitende Kontaktflächen verzichtet werden. Dies erhöht die Funktionssicherheit, erleichtert die Biokompatibilität und schließt die Korrosion solcher elektrisch leitenden Kontaktflächen aus.

In einer besonders bevorzugten Ausführungsform der Erfindung umfasst die galvanische Koppeleinrichtung zwei Elektroden am Stecker. In einer weiteren bevorzugten Ausführungsform der Erfindung umfasst die galvanische Koppeleinrichtung zwei Elektroden am Stecker, welche jedoch verschieden sind von den für Therapie und/oder Diagnostik genutzten Elektroden der Elektrodenleitung.

In einer weiteren besonders bevorzugten Ausführungsform der Erfindung ist die nichtgalvanische Koppeleinrichtung als induktive, kapazitive und/oder akustische Koppeleinrichtung ausgeführt. Als induktive Koppeleinrichtung kommt insbesondere mindestens eine Spule infrage, optional versehen mit einem Kernmaterial zur besseren Kopplung, beispielsweise Ferrit. Eine kapazitive Koppeleinrichtung umfasst bevorzugt einen Teil (bildlich eine Platte) eines Kondensators. Ein Mantel der Elektrodenleitung kann als Dielektrikum dienen. Am Implantat kann eine Koppeleinrichtung vorgesehen sein, welche zusammen mit der kapazitiven Koppeleinrichtung der Elektrodenleitung einen Kondensator bildet, wenn die Elektrodenleitung korrekt mit dem Implantat verbunden ist. Eine akustische Koppeleinrichtung ist bevorzugt als Ultraschall-Koppeleinrichtung ausgeführt. Besonders bevorzugt weist sie ein Piezoelement auf.

Auch die Auskoppeleinrichtung ist gegebenenfalls bevorzugt galvanisch oder nichtgalvanisch analog zur oben beschriebenen Koppeleinrichtung ausgeführt.

Erfindungsgemäß ist auf der Basis mindestens eines gemessenen Werts oder einer gemessenen Kennlinie der charakteristischen Größe mindestens eine individuelle Eigenschaft der Elektrodenleitung bestimmbar, beispielsweise die zugehörige Seriennummer, Losnummer der Typ, die Ausführung, Revision oder dergleichen. Die individuelle Eigenschaft stellt eine abstrakte Größe dar, welche der Elektrodenleitung vorab zugeordnet wurde, und unterscheidet sich demnach von den inhärenten elektrischen und/oder materialspezifischen Eigenschaften der Elektrodenleitung. Die individuelle Eigenschaft ist dabei nicht notwendigerweise eine einzigartige Eigenschaft. So können beispielsweise mehrere Elektrodenleitungen gemeinsam als individuelle Eigenschaft denselben Typ und dieselbe Ausführung aufweisen. Allerdings kann eine individuelle Eigenschaft auch eine einzigartige Eigenschaft darstellen, beispielsweise eine einzigartige Seriennummer einer Elektrodenleitung, wenn die entsprechenden Elektrodenleitungen einer Serie fortlaufend nummeriert werden, wobei es sich bei der Seriennummer nicht um eine Zahl handeln muss; auch Identifikationscodes anderer Art sind möglich. Beispielsweise kann eine Seriennummer Zahlen und/oder andere Zeichen umfassen.

Vorzugsweise ist der ID-Schaltkreis steckerseitig in der Elektrodenleitung angeordnet. Dadurch können die Signallaufzeiten zwischen der steckerseitig angeordneten Koppeleinrichtung und gegebenenfalls der steckerseitig angeordneten Auskoppeleinrichtung und dem ID-Schaltkreis kurz gehalten werden. Die kürzeren Signalwege reduzieren auch die Gefahr von Interferenzen mit dem Außenraum.

Eine Kennlinie kann insbesondere den Verlauf einer aus dem ID-Schaltkreis erhobenen charakteristischen Größe über der Zeit, Frequenz, der eingebrachten Anregung (z. B. Strom, Spannung) und/oder einer zweiten, charakteristischen Größe darstellen. Unter einer Kennlinie ist im Allgemeinen auch eine höherdimensionale Kennfläche zu verstehen. Der gewünschte Zahlenraum für die individuelle Eigenschaft, wie z.B. eine Seriennummer, kann über die Komplexität der Kennlinie eingestellt werden. Hierbei ist die Komplexität beispielsweise über die Anzahl der lokalen Extrema und/oder Wendepunkte definiert.

Die charakteristische Größe ist bevorzugt eine Spannung, ein Strom, eine Impedanz, eine Frequenz, eine mechanische Auslenkung und/oder ein Schalldruck. Besonders bevorzugt kann die charakteristische Größe eine Amplituden- und/oder Phaseninformation beinhalten.

In einer Weiterbildung der Erfindung werden zur Bestimmung der einen individuellen Eigenschaft mindestens ein lokales Extremum und/oder mindestens ein Wendepunkt einer Kennlinie der charakteristischen Größe und/oder deren erster und/oder höherer Ableitung bestimmt. Solche besonderen Merkmale sind gut zu identifizieren und können für eine zuverlässige Bestimmung der mindestens einen individuellen Eigenschaft genutzt werden.

Bevorzugt weist der ID-Schaltkreis mindestens einen Resonator, vorzugsweise zwei oder mehrere parallel geschaltete Resonatoren, und/oder mindestens einen nichtlinearen Spannungsteiler, vorzugsweise zwei oder mehrere kaskadisch geschaltete nichtlineare Spannungsteiler, jeweils mit einer Nichtlinearität, und/oder mindestens eine Verzögerungseinheit, vorzugsweise mehrere parallel und/oder in Reihe angeordnete Verzögerungseinheiten, auf.

Besonders bevorzugt ist der mindestens eine Resonator als LC-Schwingkreis, gegebenenfalls mit einem Dämpfungsglied, vorzugsweise in Form eines elektrischen Widerstandes, als Piezoelement, besonders bevorzugt im Ultraschallbereich, und/oder als SAW-Resonator (Surface Acoustic Wave Resonator) ausgebildet. Hierdurch lässt sich in einer frequenzabhängigen Kennlinie, insbesondere in einer frequenzabhängigen Impedanzkennlinie, eine Resonanz hervorrufen, welche als Messwert dienen kann. Die Bestimmung einer Resonanz kann besonders genau erfolgen.

Der nichtlineare Spannungsteiler weist mindestens eine Nichtlinearität auf. Er kann insbesondere so aufgebaut sein, dass einer der beiden ohmschen Widerstände eines einfachen Spannungsteilers durch eine Diode ersetzt ist. Mit einem nichtlinearen Spannungsteiler kann beispielsweise ein Wendepunkt in einer spannungsabhängigen Kennlinie einer Stromstärke erzeugt werden. In einer besonders bevorzugten Ausführungsform der erfindungsgemäßen Elektrodenleitung ist bei dem nichtlinearen Spannungsteiler der eine der beiden ohmschen Widerstände durch zwei antiparallel geschaltete Dioden ersetzt. Weiter bevorzugt beträgt die Abweichung der Kenndaten der Dioden des antiparallelen Diodenpaares, z. B. die Öffhungsspannung der Diode, weniger als 5 %. Hierbei können jeweils die Kenndaten eines Paares von Dioden annähernd gleich sein, vorzugsweise jedoch verschieden von den eines weiteren Diodenpaares. Diese Ausführungsform bietet den Vorteil, dass die Entstehung von Gleichanteilen vermieden wird. Gleichanteile sind speziell bei einer Realisierung mit Gewebekontakt bzw. mit Gewebekontaktpol unerwünscht.

Alternativ kann durch Einsatz hoher Frequenzen (vorzugsweise größer als 1 kHz) bei Gewebekontakt der Elektrodenleitung eine versehentliche Stimulation vermieden werden.

Ein erster und ein zweiter nichtlinearer Spannungsteiler können insbesondere dadurch kaskadisch geschaltet sein, dass der zweite nichtlineare Spannungsteiler parallel zum ohmschen Widerstand des ersten nichtlinearen Spannungsteilers geschaltet ist. In gleicher Art und Weise kann die kaskadische Schaltung mit weiteren nichtlinearen Spannungsteilern fortgesetzt werden.

In einer besonders vorteilhaften Ausführungsform der erfindungsgemäßen Elektrodenleitung weist jede Verzögerungseinheit einen bidirektionalen Signal-Welle-Wandler, eine Verzögerungsstrecke und einen Reflektor auf. Ein Signal ist durch den Signal-Welle-Wandler in eine Welle umwandelbar, welche sich über die Verzögerungsstrecke bis zum mit dieser verbundenen Reflektor ausbreitet und zum Signal-Welle-Wandler zurück reflektierbar ist. Dieser Ablauf nimmt eine gewisse Zeit in Anspruch. Der bidirektionale Signal-Welle-Wandler kann die zurückreflektierte Welle wieder in ein (dann verzögertes) Signal umwandeln. Je nach Ausgestaltung der Verzögerungseinheit, insbesondere der Länge der Verzögerungsstrecke und der Ausbreitungsgeschwindigkeit der Welle, lässt sich die entsprechende Verzögerungszeit einstellen. Verzögerungseinheiten können parallel und/oder in Reihe angeordnet sein. Besonders bevorzugt werden mehrere Verzögerungseinheiten parallel angeordnet. Dadurch ruft ein eingebrachtes Signal mehrere verzögerte Signale hervor. Durch unterschiedliche Einstellung der Verzögerungszeiten lassen sich mit unterschiedlich verzögerten Signalen komplexe Kennlinien generieren. Als Signal-Welle-Wandler wird beispielsweise ein Piezoelement eingesetzt, das elektrische Signale in Schall umwandelt, der dann in die Verzögerungsstrecke geleitet wird. Als Medium für die Verzögerungstrecken wird bevorzugt ein Material mit geringer Ausbreitungsgeschwindigkeit (besonders bevorzugt kleiner als 1000 m/s) verwendet, um auch bei leicht realisierbaren Pulsbreiten größer als 1 µs noch kleine Abmessungen realisieren zu können. Alternativ oder zusätzlich können die Längen von Verzögerungstrecken durch mäanderartige Strukturen verlängert werden. Die Verzögerungsstrecke kann auch durch einen elektrischen Wellenleiter realisiert werden, der insbesondere bei kürzeren Pulsbreiten (kleiner als 1 µs) vorteilhaft ist. Der Reflektor kann durch das Aufbringen einer Beschichtung und/oder durch die Nutzung einer Grenzfläche realisiert sein.

In einer weiteren vorteilhaften Ausführungsform weist die erfindungsgemäße Elektrodenleitung mindestens einen Energiespeicher auf, der mit dem ID-Schaltkreis verbunden ist, beispielsweise einen Kondensator und/oder eine Sekundärzelle. Der Energiespeicher dient besonders bevorzugt der temporären Energiespeicherung. Mithilfe des Energiespeichers kann an Nichtlinearitäten, insbesondere an Dioden, eine Vorspannung angelegt werden.

Die Realisierung des ID-Schaltkreises kann als analoge Schaltung und/oder als Kontinuum unter Nutzung charakteristischer Materialeigenschaften sowie deren räumlichen Verteilung erfolgen.

Bevorzugt ist der ID-Schaltkreis als Analogchip ausgeführt. Das ermöglicht eine einfache und kostengünstige Herstellung. Zudem kann der ID-Schaltkreis in dieser Ausführungsform sehr kompakt ausgeführt und besonders einfach in die Elektrodenleitung integriert werden.

In einer vorteilhaften Ausführungsform der Erfindung wird die Kennlinie durch den ID-Schaltkreis auf einen IEGM-Kanal aufmoduliert (IEGM = Intracardiac Electrogram). Hierfür wird vorzugsweise ein Frequenzbereich verwendet, der nahe an dem der physiologischen Signale liegt, diesen aber nicht überschneidet, z. B. ein Frequenzbereich von 1 kHz bis 10 kHz. Die Kennlinie wird bevorzugt in einen Bereich des IEGM eingemischt, der eben ist, z. B. die PQ-Strecke oder die ST-Strecke. In diesem Bereich wird ein Signalblock eingebracht, dessen Träger ein Sinus mit einer Frequenz im oben angegebenen Frequenzbereich ist. Die Amplitude dieses Signalblocks folgt der Form der zu übertragenden Kennlinie. Durch die gewählte Trägerfrequenz wird dieses nicht ins IEGM gehörende Signal von den Sensingschaltungen des Implantats, die das eigentliche IEGM-Signal überwachen, verarbeiten und ggf. übertragen, weggefiltert. Es wird jedoch in das Implantat übertragen und dort, wie oben dargestellt, analysiert.

In einer weiteren vorteilhaften Ausführungsform der Erfindung wird die digitalisierte Kennlinie des ID-Schaltkreises auf digitalem Weg mit dem digitalisierten IEGM zu einem erweiterten IEGM zusammengeführt. Die Kennlinie wird dabei bevorzugt in einen Bereich des IEGM eingebaut, der eben ist, z. B. die PQ-Strecke oder die ST-Strecke. Hierdurch wird das IEGM in den wesentlichen Teilen nicht modifiziert. Grundsätzlich kann dieser Einbau jedoch an jeder Stelle des IEGM erfolgen. Anfang und Ende der Sequenz mit der digitalisierten Kennlinie des ID-Schaltkreises werden bei der Zusammenführung gekennzeichnet. Das erweiterte IEGM kann vom abfragenden Implantat an ein weiteres Gerät, z. B. ein Patientengerät oder ein Programmiergerät übertragen werden. Das weitere Gerät kann anhand der Kennzeichnung von Anfang und Ende der Sequenz mit der digitalisierten Kennlinie des ID-Schaltkreises diese Sequenz aus dem erweiterten IEGM herauslösen. Beide Komponenten, nämlich das digitalisierte IEGM und die digitalisierte Kennlinie des ID-Schaltkreises, können anschließend vom weiteren Gerät getrennt analysiert werden. Hierdurch erfolgt die für die Identifikation notwendige Auswertung im weiteren Gerät und nicht im Implantat.

Eine weitere Aufgabe der vorliegenden Erfindung besteht darin, ein einfaches und implantationsunabhängiges Verfahren zur Identifikation einer Elektrodenleitung mit den Merkmalen des Anspruchs 8 anzugeben.

Die Aufgabe wird gelöst durch ein Verfahren zur Identifikation einer erfindungsgemäßen Elektrodenleitung, umfassend die folgenden Schritte, vorzugsweise in der unten stehenden Reihenfolge:
- Anregen des ID-Schaltkreises über die Koppeleinrichtung,
- Messen mindestens einer Kennlinie der charakteristischen Größe des ID-Schaltkreises während oder nach der Anregung,
- Bestimmen mindestens einer individuellen Eigenschaft der Elektrodenleitung, beispielsweise die zugehörige Seriennummer, Losnummer, Typ, Ausführung, Revision oder dergl., basierend auf der mindestens einen gemessenen Kennlinie.

Das erfindungsgemäße Verfahren erlaubt es auf einfache, kostengünstige und schnelle Weise, als mindestens eine individuelle Eigenschaft einer geeigneten Elektrodenleitung beispielsweise deren Seriennummer und/oder deren Typ zu bestimmen. Mit dem Verfahren kann eine Vorrichtung, an die eine geeignete Elektrodenleitung mit ID-Schaltkreis angeschlossen ist, feststellen, ob die richtige Elektrodenleitung angeschlossen ist und/oder ob sie für die vorgesehene Verwendung geeignet ist. Das Verfahren kann auch zur Unterscheidung von Elektrodenleitungen anhand ihrer mindestens einen individuellen Eigenschaft dienen. Dies beugt der Verwendung nicht geeigneter Elektroden vor, erhöht die Verwendungssicherheit und schützt die Gesundheit von Patienten. Der Vorteil des erfindungsgemäßen Verfahrens liegt weiter darin, dass der ID-Schaltkreis bedarfsgerecht angeregt werden kann, wodurch Energie gespart werden kann. Der ID-Schaltkreis ist deshalb nicht auf eine dauerhafte Energieversorgung angewiesen.

Anhand der mindestens einen individuellen Eigenschaft, insbesondere einer Seriennummer, können optional weitere individuelle Eigenschaften der Elektrodenleitung ermittelt werden, beispielsweise die Art und/oder der Typ der Elektrodenleitung, die vorgesehene Verwendung, ein vorgesehener Einsatzort, der Herstellungszeitpunkt, die maximale Nutzungsdauer, eine bestimmte elektrische Eigenschaft, eine Elektrodenanzahl, die Art und/oder der Typ angeschlossener Elektroden, eine Elektrodenkonfiguration, die Anzahl therapeutischer und/oder diagnostischer Leiter, die Länge und/oder der Querschnitt therapeutischer Leiter, Informationen zur Ansteuerung oder dergleichen. Besonders bevorzugt können solche weiteren individuellen Eigenschaften aus einer Speichereinrichtung, vorzugsweise mit einer Datenbank, entnommen werden.

Bevorzugt erfolgt die Messung mindestens eines Wertes oder einer Kennlinie einer charakteristischen Größe des ID-Schaltkreises analog. Dadurch kann auf zusätzliche, digitale Messtechnik bei der Messung der charakteristischen Größe des ID-Schaltkreises verzichtet werden. Dies schließt nicht aus, dass die analoge Messung und/oder die weiterverarbeitete Messung außerhalb des ID-Schaltkreises diskretisiert und/oder digitalisiert wird. Das erleichtert die weitere Bearbeitung, Übertragung und Auswertung der Messung.

In einer vorteilhaften Weiterbildung des Verfahrens umfasst das Bestimmen der mindestens einen individuellen Eigenschaft folgende weitere Schritte:
- Bestimmen mindestens eines lokalen Extremums und/oder mindestens eines Wendepunkts der gemessenen Kennlinie der charakteristischen Größe und/oder deren erster und/oder höherer Ableitung der Kennlinie,
- Ermitteln eines Codes der individuellen Eigenschaft auf Basis des bestimmten mindestens einen Extremums und/oder mindestens einen Wendepunkts.

Dadurch können gezielt besondere Merkmale der beschriebenen Elektrodenleitung bzw. des ID-Schaltkreises zur Bestimmung der individuellen Eigenschaft genutzt werden.

Besonders bevorzugt werden zur Ermittlung des Codes Metriken eingesetzt, die die Koordinate (Abszisse und Ordinate, gegebenenfalls auch mehrdimensional) des bestimmten mindestens einen Extremums und/oder mindestens einen Wendepunkts der gemessenen Kennlinie verwenden. Darauf basierend wird mindestens eine Kennzahl ermittelt. Die Zahlenbasis der Kennzahl ist durch die Anzahl der Diskretisierungsstufen gegeben. Der Code besteht aus allen ermittelten Kennzahlen. Der gewünschte Zahlenraum für den Code ist über die Komplexität der Kennlinie, insbesondere über die Anzahl der lokalen Extrema und/oder Wendepunkte der gemessenen Kennlinie und/oder ihrer ersten und/oder ihrer höheren Ableitungen sowie die Feinheit der Diskretisierung einstellbar. Hierbei können bezüglich unterschiedlicher Kennzahlen unterschiedliche Diskretisierungsstufen genutzt werden.

Dafür kann die gemessene Kennlinie in mindestens zwei Abschnitte oder Diskretisierungsfelder unterteilt werden. Die Abschnitte müssen nicht aneinander angrenzen und insgesamt nicht die komplette Kennlinie abdecken. Die Kennzahl(en) des bestimmten mindestens einen Extremums und/oder mindestens einen Wendepunkts sind in diesem Fall dadurch gegeben, in welchem Abschnitt und/oder Diskretisierungsfeld das Extremum und/oder der Wendepunkt liegt.

Besonders bevorzugt wird die mindestens eine Kennzahl in einer durch eine Diskretisierung gegebenen Zahlenbasis gebildet und anschließend in eine andere Zahlenbasis umgerechnet. Dadurch kann der Code in eine gewünschte Darstellung gebracht werden.

In einer weiteren vorteilhaften Weiterbildung des Verfahrens umfasst das Bestimmen der mindestens einen individuellen Eigenschaft folgende weitere Schritte:
- Vergleichen der gemessenen Kennlinie der charakteristischen Größe mit mindestens einer gespeicherten Kennlinie,
- Ermitteln eines Codes der individuellen Eigenschaft auf der Basis des Ergebnisses des Vergleichs.

Diese Weiterbildung bietet den Vorteil, dass Informationen der gesamten gemessenen Kennlinie berücksichtigt werden. Insbesondere wird auch der Verlauf der gemessenen Kennlinie außerhalb der Bereiche, in denen Extrema oder Wendepunkte von ihr und/oder ihrer ersten und/oder ihrer höheren Ableitung liegen, mit ausgewertet. Dadurch wird das Bestimmen der mindestens einen individuellen Eigenschaft unempfindlicher gegen unerwartete Veränderungen und/oder Abweichungen von lokalen Extrema und/oder Wendepunkten. So etwas könnte beispielsweise dann auftreten, wenn sich die Resonanz eines Resonators verschieben würde.

Darüber hinaus ist es Aufgabe der Erfindung, ein Implantat: mit den Merkmalen des Anspruchs 11 zur Verfügung zu stellen, mit dem eine erfindungsgemäße Elektrodenleitung einfach und ohne zusätzlichen Aufwand identifiziert werden kann.

Die Aufgabe wird gelöst durch ein Implantat mit einer Buchse zur Verbindung mit dem Stecker einer oben erwähnten Elektrodenleitung, mit einer an oder in der Buchse angeordneten Koppeleinrichtung sowie einer Identifizierungseinrichtung, wobei die Identifizierungseinrichtung derart ausgestaltet ist, dass mittels der Koppeleinrichtung des Implantats über die Koppeleinrichtung der Elektrodenleitung der ID-Schaltkreis der Elektrodenleitung anregbar, nach oder während der Anregung eine charakteristische Größe des ID-Schaltkreises über die Koppeleinrichtung der Elektrodenleitung und des Implantats und/oder über die Auskoppeleinrichtung der Elektrodenleitung messbar ist und dass auf der Basis mindestens einer gemessenen Kennlinie der charakteristischen Größe mindestens eine individuelle Eigenschaft der Elektrodenleitung, beispielsweise die zugehörige Seriennummer, Losnummer, Typ, Ausführung, Revision oder dergleichen, bestimmbar ist.

Das Implantat erlaubt es analog zum oben erläuterten erfindungsgemäßen Verfahren mit den oben erwähnten Vorteilen, mindestens eine individuelle Eigenschaft einer geeigneten Elektrodenleitung, beispielsweise deren Seriennummer und/oder deren Typ zu bestimmen. So kann festgestellt werden, ob die richtige oder welche Elektrodenleitung an einer bestimmten Buchse des Implantats angeschlossen ist. Ein weiterer Vorteil des erfindungsgemäßen Implantats liegt darin, dass es den ID-Schaltkreis anregen kann und deshalb dieser nicht auf eine dauerhafte Energieversorgung angewiesen ist. Weiterhin findet die Kopplung zwischen Implantat und ID-Schaltkreis bedarfsgerecht statt, so dass Energie gespart wird, welche aus dem Energiespeicher des Implantats zur Verfügung gestellt werden muss.

Ein weiterer Vorteil des erfindungsgemäßen Implantats ist, dass es die Nutzung und Ansteuerung der Elektrodenleitung gegebenenfalls anhand der Bestimmung ihrer mindestens einen individuellen Eigenschaft anpassen kann. Das erlaubt einen sichereren, effektiveren Einsatz des Implantats sowie der Elektrodenleitung und verbessert den Behandlungserfolg.

In einer bevorzugten Ausführungsform des erfindungsgemäßen Implantats bestimmt die Identifizierungseinrichtung analog zum oben beschriebenen Verfahren einen Code der individuellen Eigenschaft. Der Code kann vorzugsweise einer Seriennummer der Elektrodenleitung entsprechen.

In einer besonders bevorzugten Weiterbildung der Erfindung bestimmt die Identifizierungseinrichtung analog zum oben erläuterten Verfahren mit den oben diskutierten Vorteilen mindestens ein lokales Extremum und/oder mindestens einen Wendepunkt der gemessenen Kennlinie der charakteristischen Größe und/oder deren erster und/oder höherer Ableitung der Kennlinie und ermittelt den Code der individuellen Eigenschaft auf Basis des bestimmten mindestens einen Extremums und/oder mindestens einen Wendepunkts.

In einer weiteren besonders bevorzugten Weiterbildung der Erfindung vergleicht die Identifizierungseinrichtung die gemessene Kennlinie der charakteristischen Größe mit mindestens einer gespeicherten Kennlinie und ermittelt den Code der individuellen Eigenschaft auf der Basis des Ergebnisses des Vergleichs. Die Vorteile dieser Vorgehensweise wurden oben zu dem Verfahren bereits erläutert.

Bevorzugt weist die Identifizierungseinrichtung eine Steuereinheit, eine Spannungsquelle, und die Koppeleinrichtung auf, die in Reihe geschaltet sind. Die über die Koppeleinrichtungen ermittelten Messungen werden der Steuereinheit übermittelt.

Besonders bevorzugt weist die Identifizierungseinrichtung zwischen der Koppeleinrichtung und der Steuereinheit weiter einen Strom-Spannungswandler, einen AD-Wandler, einen Demodulator und/oder einen Peak-Detektor zur Weiterverarbeitung der Messung auf. Ferner ist eine Speichereinheit vorgesehen, in der die ermittelten Daten zur individuellen Eigenschaft der Elektrodenleitung abgelegt werden können. Die Speichereinheit kann auch mit der Identifizierungseinheit verbunden sein.

In einer alternativen Ausführungsform des erfindungsgemäßen Implantats wird die gemessene Kennlinie ohne Weiterverarbeitung gesampelt und aus dem Implantat an eine externe Dekodiereinheit geschickt, welche bevorzugt in einer Programmier- oder Homemonitoring-Vorrichtung enthalten ist. Die Sampelwerte werden in diesem Fall direkt in die Speichereinheit hinein geschrieben. Die noch fehlenden Auswertungsschritte werden extern in der Dekodiereinheit ausgeführt.

Eine weitere alternative Möglichkeit der Auswertung der gemessenen Kennlinie zur Ermittlung der individuellen Eigenschaft der Elektrodenleitung besteht darin, die Kennlinie mit in der Speichereinheit, welche als Datenbank ausgestaltet sein kann, gespeicherten Kennlinien mittels der Methode der kleinsten Fehlerquadrate zu vergleichen. Durch den Vergleich wird die gespeicherte nächstliegende Kennlinie ermittelt, welche der gemessenen Kennlinie am besten entspricht, d.h. den kleinsten Fehlerquadratwert aufweist. Vorzugsweise ist jeder gespeicherten Kennlinie in der Speichereinheit ein Code zugeordnet. Der Code der Elektrodenleitung ergibt sich nun aus dem zugeordneten Code der nächstliegenden Kennlinie.

Der Frequenzbereich zur Anregung des ID-Schaltkreises und Bestimmung der charakteristischen Größe durch das erfindungsgemäße Implantat bzw. die Identifizierungseinrichtung liegt bevorzugt zwischen 10 Hz und 1 GHz, besonders bevorzugt zwischen 10 Hz und 150 kHz.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: ein erstes und ein zweites Ausführungsbeispiel der erfindungsgemäßen Elektrodenleitung, die jeweils mittels eines Steckers mit einem erfindungsgemäßen Implantat verbunden sind, in einem Querschnitt,
- Fig. 2: ein Schaltbild mit einem ersten Ausführungsbeispiel eines ID-Schaltkreises des ersten Ausführungsbeispiels der Elektrodenleitung gemäß Fig. 1 aufweisend vier parallel geschaltete Resonatoren und mit einer Koppeleinrichtung,
- Fig. 3: eine Kennlinie der Impedanz abhängig von der Stromstärke des ID-Schaltkreises gemäß Fig. 2,
- Fig. 4: ein Schaltbild mit einem zweiten Ausführungsbeispiel eines ID-Schaltkreises des ersten Ausführungsbeispiels der Elektrodenleitung gemäß Fig. 1 aufweisend drei kaskadisch geschaltete, nichtlineare Spannungsteiler und mit der Koppeleinrichtung,
- Fig. 5: eine Kennlinie der Stromstärke abhängig von der Spannung des ID-Schaltkreises gemäß Fig. 4,
- Fig. 6: ein Schaltbild mit einem dritten Ausführungsbeispiel des ID-Schaltkreises des ersten Ausführungsbeispiels der Elektrodenleitung gemäß Fig. 1 aufweisend vier parallel angeordnete Verzögerungseinheiten und mit der Koppeleinrichtung,
- Fig. 7: eine Prinzipdarstellung einer teilweise diskretisierten Kennlinie des ID-Schaltkreises einer erfindungsgemäßen Elektrodenleitung,
- Fig. 8: ein Schaltbild mit einem ersten Ausführungsbeispiel einer Identifizierungseinrichtung, mit der nichtgalvanischen Koppeleinrichtung des Implantats gemäß Fig. 1, mit der Koppeleinrichtung und mit dem ID-Schaltkreis gemäß Fig. 2 und
- Fig. 9: ein Schaltbild mit einem zweiten Ausführungsbeispiel einer Identifizierungseinrichtung, der nichtgalvanischen Koppeleinrichtung des Implantats gemäß Fig. 1, mit der Koppeleinrichtung und dem ID-Schaltkreis gemäß Fig. 6.

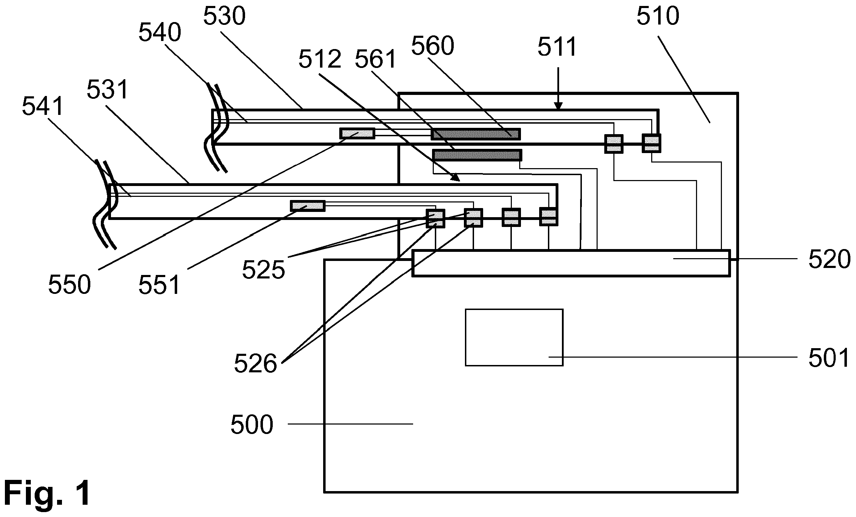

Fig. 1 zeigt ein erstes Ausführungsbeispiel der erfindungsgemäßen Elektrodenleitung (erste Elektrodenleitung 530) und ein zweites Ausführungsbeispiel einer erfindungsgemäßen Elektrodenleitung (zweite Elektrodenleitung 531) sowie ein erfindungsgemäßes Implantat 500, zum Beispiel einen Herzschrittmacher, Neurostimulator oder Defibrillator. Die erste und die zweite Elektrodenleitung 530, 531 sind jeweils mittels eines Steckers 511, 512 mit dem Implantat 500 verbunden und weisen jeweils mindestens einen elektrischen (therapeutischen oder diagnostischen) Leiter 540, 541 auf. Des Weiteren enthalten die erste und die zweite Elektrodenleitung 530, 531 jeweils einen steckerseitig angeordneten ID-Schaltkreis 550, 551 und jeweils eine am Stecker vorgesehene Koppeleinrichtung 560, 525. Jeder ID-Schaltkreis 550, 551 ist mit der zugehörigen Koppeleinrichtung 560, 525 elektrisch verbunden. Mit dem jeweiligen therapeutischen oder diagnostischen Leiter 540, 541 ist der ID-Schaltkreis 550, 551 nicht elektrisch verbunden.

Über eine im Bereich einer Buchse vorgesehene nichtgalvanische Koppeleinrichtung 561 eines Implantats 500, wobei die Buchse im Header 510 des Implantats 500 angeordnet ist, und die nichtgalvanische Koppeleinrichtung 560 der Elektrodenleitung 530 kann der ID-Schaltkreis 550 der Elektrodenleitung 530 beispielsweise induktiv angeregt werden. Hierfür sind die Koppeleinrichtungen 560, 561 als Spulen ausgestaltet, welche zur Erhöhung der magnetischen Flussdichte vorzugsweise einen Ferritkern aufweisen. Nach der Anregung wird über die Koppeleinrichtung 560 eine charakteristische Größe des ID-Schaltkreises 550 vom Implantat 500 über dessen Koppeleinrichtung 561 gemessen. Die Koppeleinrichtung 561 des Implantats wird dazu von einer in dem Implantat 500 angeordneten Identifizierungseinrichtung 501 angesteuert und mit Energie versorgt.

Wenn die zweite Elektrodenleitung 531 mittels ihres Steckers 512 mit dem Implantat 500 verbunden ist, berühren sich die elektrischen Kontakte der galvanischen Koppeleinrichtung 525 der Elektrodenleitung und die elektrischen Kontakte einer an der Buchse vorgesehenen galvanischen Koppeleinrichtung 526 des Implantats, sodass sie elektronisch leitend verbunden sind. Dadurch kann der ID-Schaltkreis 551 der zweiten Elektrodenleitung 531 elektrisch angeregt werden. Nach der Anregung wird über die Koppeleinrichtungen 525, 526 eine charakteristische Größe des ID-Schaltkreises 551 gemessen. Die Koppeleinrichtungen 561, 526 in dem Header 510 sind jeweils elektrisch leitend über eine Durchführung 520 mit dem Implantat 500 verbunden.

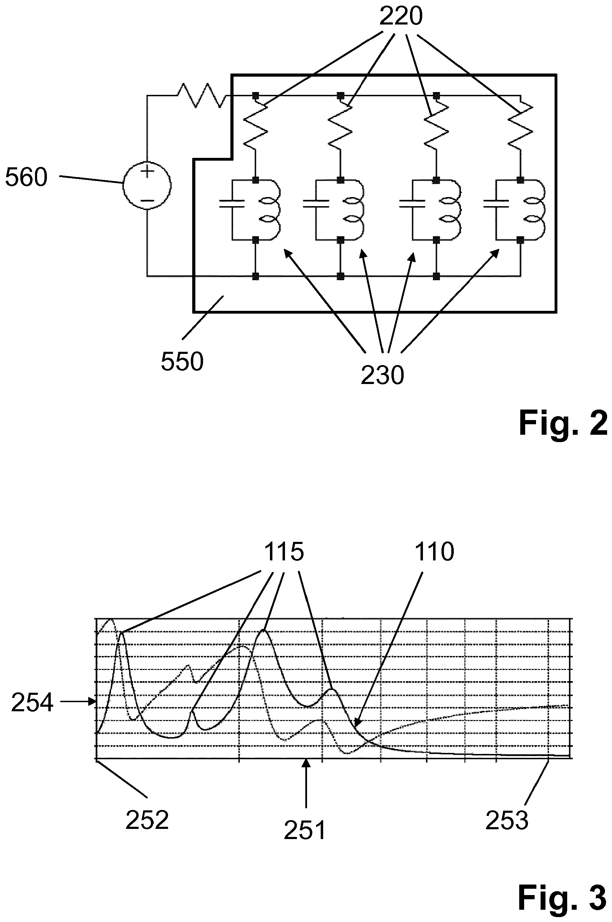

Das in Fig. 2 gezeigte erste Ausführungsbeispiel eines ID-Schaltkreises 550 weist vier parallel geschaltete Resonatoren in Form von LC-Schwingkreisen 230 auf. Zu jedem LC-Schwingkreis 230 ist zusätzlich ein elektrischer Widerstand 220 in Reihe geschaltet. Als charakteristische Größe wird bei diesem Ausführungsbeispiel die Impedanz 254 in Abhängigkeit von der Frequenz 251 über die Koppeleinrichtungen 560, 561 gemessen. Eine entsprechende Kennlinie 110 ist in Fig. 3 dargestellt.

Die Kennlinie 110 weist mehrere lokale Maxima bzw. Peaks 115 der Impedanz 254 auf, die in einem Frequenzbereich, der von einer unteren Frequenzgrenze 252 bei 50 MHz bis zu einer oberen Frequenzgrenze 253 bei 450 MHz reicht, liegen. Da jedes lokale Maximum 115 jeweils durch einen Resonator des ID-Schaltkreises 550 verursacht wird, kann die Lage und die Anzahl der Maxima durch die Dimensionierung der Resonatoren bestimmt werden. Anhand der Maxima wird, wie unten erläutert wird, die individuelle Eigenschaft der ersten Elektrodenleitung 530 bestimmt.

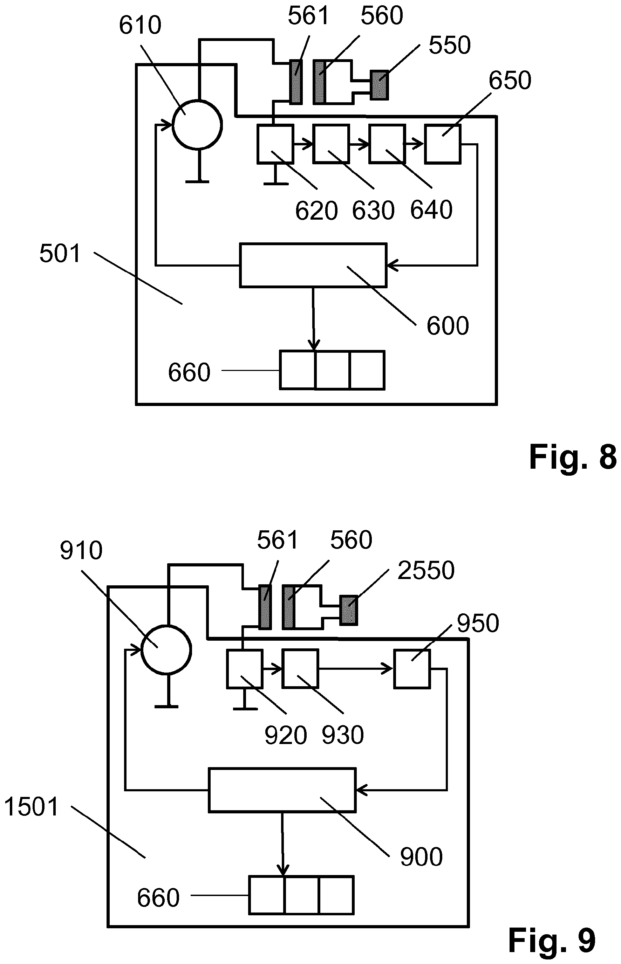

Es soll an dieser Stelle darauf hingewiesen werden, dass die Impedanz 254 in Abhängigkeit von der Frequenz des ID-Schaltkreises 550 auch, wie zu Fig. 8 beschrieben, anhand der Messung einer Kennlinie 110, die abhängig von der Zeit ist, ermittelt werden kann.

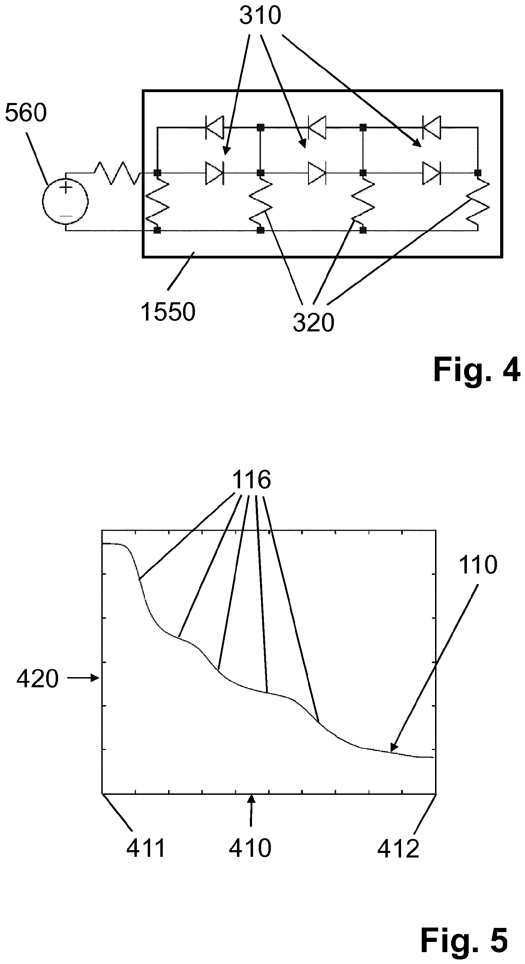

Das in Fig. 4 gezeigte zweite Ausführungsbeispiel eines ID-Schaltkreises 1550 weist drei kaskadisch geschaltete, nichtlineare Spannungsteiler auf, wobei jeder nichtlineare Spannungsteiler durch eine Reihenschaltung von einem ohmschen Widerstand 320 und einem Paar von antiparallel geschalteten Dioden 310 (Nichtlinearitäten) gebildet ist. Bevorzugt beträgt die Abweichung der Kenndaten, z. B. die Öffnungsspannung, der Dioden eines antiparallelen Diodenpaares 310 weniger als 5 %. Als charakteristische Größe wird zu diesem ID-Schaltkreis 1550 die Stromstärke 420 in Abhängigkeit von der Spannung 410 gemessen. Ein Beispiel einer Kennlinie 110 ist in Fig. 5 für eine gleichbleibende Frequenz gezeigt. Die Kennlinie 110 zeigt im Bereich zwischen 0 V (untere Spannungsgrenze 411) und 5 V (obere Spannungsgrenze 412) vier Wendepunkte 116, deren Lage und Anzahl durch die Dimensionierung der nichtlinearen Spannungsteiler erzeugt und die zur Bestimmung der individuellen Eigenschaft der ersten Elektrodenleitung herangezogen werden.

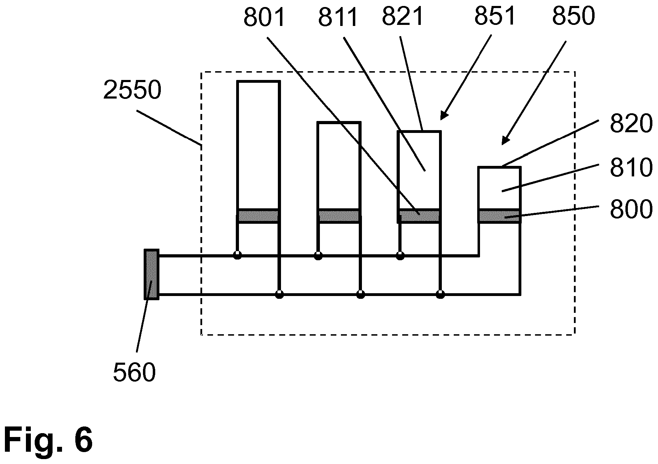

Ein drittes Ausführungsbeispiel eines ID-Schaltkreises 2550 mit vier parallel zueinander geschalteten Verzögerungseinheiten 850, 851, die jeweils einen bidirektionalen Signal-Welle-Wandler 800, 801, eine Verzögerungsstrecke 810, 811 und einen als Beschichtung aufgebrachten Reflektor 820, 821 aufweisen, ist in Fig. 6 dargestellt.

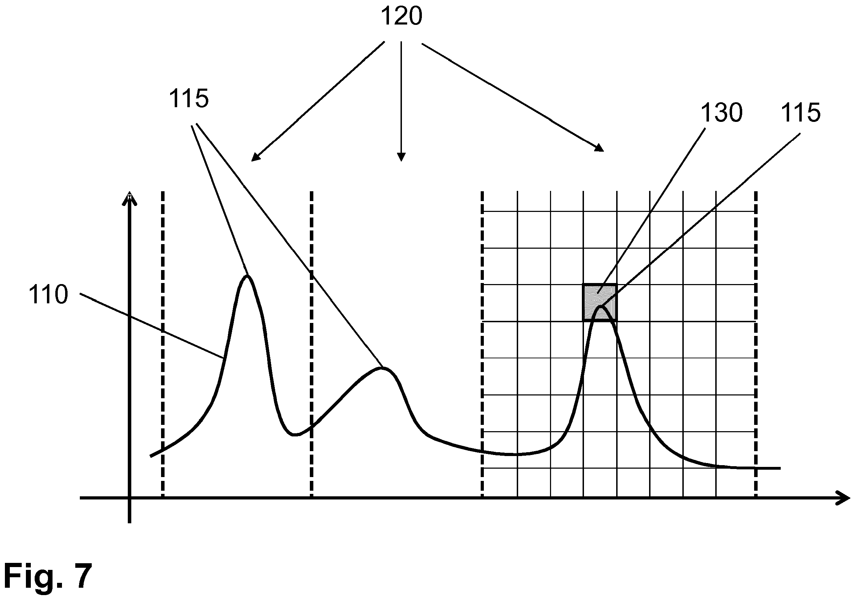

Eine in die Koppeleinrichtung 560 eingekoppelte Anregung wird in dem ID-Schaltkreis 2550 praktisch verzögerungsfrei an jeden Signal-Welle-Wandler in Form eines Piezoelements 800, 801 geleitet. Die jeweils von dort ausgehende Welle breitet sich entlang der zugehörigen Verzögerungsstrecke 810, 811 aus, wird an dem Reflektor 820, 821 reflektiert und gelangt an den Signal-Welle-Wandler 800, 801 zurück. Die Verzögerungszeit ergibt sich aus der Ausbreitungsgeschwindigkeit der Welle im Medium der jeweiligen Verzögerungsstrecke 810, 811 und deren Länge. Nach Rückwandlung der Welle in ein verzögertes Signal kann dieses über die Koppeleinrichtung 560 gemessen werden. Die verzögerten Signale der Verzögerungseinheiten 850, 851 überlagern sich und ergeben eine zeitabhängige Kennlinie 110, die unten anhand von Fig. 7 erläutert wird.

Mit Bezug zu Fig. 7 werden nun drei Möglichkeiten zur Bestimmung der mindestens einen individuellen Eigenschaft der ersten oder zweiten Elektrodenleitung 530, 531 als Beispiele für ein erfindungsgemäßes Verfahren zur Identifikation einer Elektrodenleitung erläutert.

Bei der in Fig. 3 dargestellten Kennlinie 110 wird, wie in Fig. 7 veranschaulicht, in jedem vorgegebenen Frequenzabschnitt 120 eine Trennung in Diskretisierungsfelder vorgenommen, wobei jeder Frequenzabschnitt 120 in Frequenzrichtung (entlang der Abszisse) in N Unterabschnitte und hinsichtlich der Amplituden in M Unterabschnitte aufgeteilt wird (siehe karierter Bereich), so dass in jedem Frequenzabschnitt N^{∗}M Diskretisierungsfelder entstehen. Um die individuelle Eigenschaft zu bestimmen, wird nun ermittelt, in welchem Diskretisierungsfeld und in welchem Frequenzabschnitt 120 ein lokales Maximum 115 liegt. Ein solches Diskretisierungsfeld ist in Fig. 7 mit dem Bezugszeichen 130 versehen. Der Index des Felds 130 innerhalb der Matrix N^{∗}M ergibt eine Kennzahl für den jeweiligen Frequenzabschnitt 120. Die Zahlenbasis N^{∗}M ist damit durch die Anzahl der Diskretisierungsstufen gegeben. Aus den Kennzahlen für alle drei Frequenzabschnitte 120 wird dann ein Code der individuellen Eigenschaft der ersten Elektrodenleitung 530 bestimmt.

Bei einer zweiten Möglichkeit, die beispielsweise für die in Fig. 5 dargestellte Kennlinie geeignet ist, wird jeder Spannungsabschnitt 120 der Fig. 7 entlang der Abszisse in M Diskretisierungsfelder unterteilt. Die für jeden Wendepunkt ermittelte Kennzahl der Basis M dient zur Generierung eines Codes, der die individuelle Eigenschaft des ID-Schaltkreises 1550 und damit der zugehörigen Elektrodenleitung 530 beschreibt. Alternativ können in dem Implantat Musterkennlinien gespeichert sein, wobei mittels der bekannten Methode der kleinsten Fehlerquadrate ermittelt wird, welche der Musterkennlinien der gemessenen Kennlinie am ähnlichsten ist. Über die identifizierte Musterkennlinie wird der vermessenen Elektrodenleitung ein entsprechender Code, z. B. über die Adresse in der Datenbank, zugeordnet.

Analog kann bei dem in Fig. 6 dargestellten dritten Ausführungsbeispiel des ID-Schaltkreises 2550 vorgegangen werden. Die gemessene Kennlinie dieses ID-Schaltkreises 2550 beinhaltet ein der zurückreflektierten Welle entsprechendes Spannungssignal (alternativ auch ein Stromsignal) in Abhängigkeit von der Zeit. Folglich entsprechen die Abschnitte 120 in Fig. 7 Zeitabschnitten. Für jeden Abschnitt 120 wird eine der Lage des Maximums des Signals auf der Abszisse in der Basis M der Diskretisierung entsprechende Kennzahl bestimmt, wobei jedes Maximum eine von einer Verzögerungseinheit zurück reflektierte Welle beschreibt. Aus den entsprechend ermittelten vier Kennzahlen wird anschließend der Code der individuellen Eigenschaft der Elektrodenleitung ermittelt.

Es soll an dieser Stelle darauf hingewiesen werden, dass es von Vorteil ist, die Längen der Verzögerungsstrecken 810, 811 verschiedener ID-Schaltkreise 2550 gemäß Fig. 6 derart zu variieren, dass in vier Zeitabschnitten 120, die für alle ID-Schaltkreise gleich sind und sich nicht überlappen, jeweils genau ein verzögertes Signal auftritt. Hierdurch kann jedes Maximum genau einer Verzögerungseinheit zugeordnet werden und die Bestimmung der individuellen Eigenschaft der Elektrodenleitung ist weniger fehleranfällig.

Fig. 8 zeigt den Aufbau der Identifizierungseinrichtung 501 des Implantats 500 aus Fig. 1 mit einer Steuereinheit 600, welche einer Sinusspannungsquelle 610 eine Frequenz für die Anregung des ID-Schaltkreises 550 zur Ermittlung der in Fig. 3 gezeigten Kennlinie 110 vorgibt. Die Amplitude ist konstant. Als charakteristische Größe des ID-Schaltkreises 550 wird ein sich an der Koppeleinrichtung 561 des Implantats einstellender Strom gemessen. Der sich einstellende Strom wird optional durch einen Strom-Spannungswandler 620 in eine Messspannung gewandelt, die vom AD-Wandler 630 digitalisiert wird. Ein Demodulator 640 bestimmt die Einhüllende des Messsignals. Aus dieser extrahiert ein Peak-Detektor 650 die lokalen Maxima und übermittelt diese der Steuereinheit 600. Die Steuereinheit 600 ermittelt daraus, wie oben anhand von Fig. 7 erläutert wurde, die individuelle Eigenschaft der ersten Elektrodenleitung 530, beispielsweise einen Code für die individuelle Eigenschaft der Elektrodenleitung 530, der in der Speichereinheit 660 abgelegt wird.

Alternativ regt die Identifizierungseinrichtung den ID-Schaltkreis 550 mit einer Impuls-oder Sprungfunktion, vorzugsweise mit einem bandbegrenzten Spannungssignal wie einer sinc-Funktion, an und misst als charakteristische Größe die Kennlinie 110 des sich einstellenden Stroms in Abhängigkeit von der Zeit. Die Fouriertransformierte der Kennlinie 110 wird durch die Fouriertransformierte der Impuls- oder Sprungfunktion dividiert, wobei die resultierende Kennlinie in Abhängigkeit von der Frequenz wie zuvor beschrieben zur Bestimmung der individuellen Eigenschaft der Elektrodenleitung verwendet wird. Durch Fouriertransformation der Impulsantwort wird der Frequenzgang, also eine Kennlinie in Abhängigkeit von der Frequenz, ermittelt.

Alternativ kann die Sprungantwort des ersten Ausführungsbeispiels des ID-Schaltkreises 550 gemessen werden. Dabei wird eine Sprungfunktion zur Anregung genutzt. Die Fouriertransformierte der Sprungantwort wird durch die Fouriertransformierte der Sprungfunktion dividiert, um den gewünschten Frequenzgang des ID-Schaltkreises 550 zu erhalten. Bevorzugt ist mit einem praktisch leichter realisierbaren bandbegrenzten Signal anzuregen (z. B. ein sinc-Puls), dessen Frequenzgang ebenso aus dem Frequenzgang der gemessenen Sprungantwort herauszurechnen ist.

Alternativ kann der Demodulator 640 auch analogseitig vor dem AD-Wandler 630 angeordnet sein. Eine derartige Gestaltung der Identifizierungseinrichtung 501 ist vorteilhaft, da der AD-Wandler 630 dann wesentlich langsamer sein darf und energiesparender arbeitet.

Analog kann auch die in Fig. 5 dargestellte Strom-Spannungskennlinie mittels der in Fig. 8 dargestellten Identifizierungseinrichtung 501 ermittelt und zur Bestimmung der individuellen Eigenschaft der ersten Elektrodenleitung 530 herangezogen werden. Da mittels der Kennlinie 110 Wendepunkte bestimmt werden, kann der Peak-Detektor 650 entfallen.

Fig. 9 veranschaulicht ein zweites Ausführungsbeispiel einer Identifizierungseinrichtung 1501 für die Bestimmung der individuellen Eigenschaft der ersten Elektrodenleitung 530 anhand des in Fig. 6 dargestellten ID-Schaltkreises 2550. Eine Steuereinheit 900 veranlasst die Anregung des ID-Schaltkreises 2550 mittels einer Pulsspannungsquelle 910 über die nichtgalvanische Koppeleinrichtung 561. Anschließend wird das mindestens eine, verzögerte Signal über die Koppeleinrichtungen 560 der ersten Elektrodenleitung 530 und die Koppeleinrichtung 561 des Implantats 500 gemessen. Das Messsignal wird nach einer Strom-Spannungs-Wandlung durch einen Strom-Spannungswandler 920 mittels AD-Wandler 930 digitalisiert. Der Peak-Detektor 950 übermittelt der Steuereinheit 900 die Verzögerungszeiten der verzögerten Signale. Hieraus wird, wie anhand von Fig. 7 oben erläutert, die charakteristische Eigenschaft der ersten Elektrodenleitung 530 in Form eines Codes ermittelt und in der Speichereinheit 660 abgelegt.

### Bezugszeichenliste

- 110: Kennlinie
- 115: lokales Maximum
- 120: Abschnitt
- 130: Diskretisierungsfeld
- 220: elektrischer Widerstand
- 230: LC-Schwingkreis
- 251: Frequenz
- 252: untere Frequenzgrenze
- 253: obere Frequenzgrenze
- 254: Impedanz
- 310: antiparalleles Diodenpaar
- 320: ohmscher Widerstand
- 410: Spannung
- 411: untere Spannungsgrenze
- 412: obere Spannungsgrenze
- 420: Stromstärke
- 500: Implantat
- 501, 1501: Identifizierungseinrichtung
- 510: Header
- 511,512: Stecker
- 520: Durchführung
- 530: erste Elektrodenleitung
- 531: zweite Elektrodenleitung
- 540, 541: therapeutischer oder diagnostischer Leiter
- 525: galvanische Koppeleinrichtung
- 526: galvanische Koppeleinrichtung des Implantats 500
- 550, 551, 1550, 2550: ID-Schaltkreis
- 560: nichtgalvanische Koppeleinrichtung
- 561: nichtgalvanische Koppeleinrichtung des Implantats 500
- 600: Steuereinheit
- 610: Sinusspannungsquelle
- 620: Strom-Spannungswandler
- 630: AD-Wandler
- 640: Demodulator
- 650: Peak-Detektor
- 660: Speichereinheit
- 800, 801: Piezoelement
- 810, 811: Verzögerungsstrecke
- 820, 821: Reflektor
- 850, 851: Verzögerungseinheit
- 900: Steuereinheit
- 910: Pulsspannungsquelle
- 920: Strom-Spannungswandler
- 930: AD-Wandler
- 950: Peak-Detektor

## Patentansprüche

1. Elektrodenleitung (530, 531) zur Verbindung mit einem Implantat (500) mittels eines Steckers (511, 512),
**gekennzeichnet durch** einen analogen elektrischen ID-Schaltkreis (550, 551), der in der Elektrodenleitung (530, 531) steckerseitig angeordnet und mit einer steckerseitig angeordneten Koppeleinrichtung (525, 560) der Elektrodenleitung (530, 531) verbunden ist,
wobei der ID-Schaltkreis (550, 551, 1550, 2550) über die Koppeleinrichtung (525, 560) anregbar und derart ausgestaltet ist, dass während oder nach der Anregung eine charakteristische Größe des ID-Schaltkreises (550, 551, 1550, 2550) über die Koppeleinrichtung (525, 560) und/oder über eine mit dem ID-Schaltkreis (550, 551, 1550, 2550) verbundene, steckerseitig angeordnete Auskoppeleinrichtung der Elektrodenleitung (530, 531) messbar ist und dass auf der Basis mindestens einer gemessenen Kennlinie (110) der charakteristischen Größe mindestens eine individuelle Eigenschaft der Elektrodenleitung (530, 531), bestimmbar ist.

2. Elektrodenleitung (530, 531) nach Anspruch 1, **dadurch gekennzeichnet, dass** die charakteristische Größe eine Spannung , ein Strom (420), eine Impedanz (254), eine Frequenz, eine mechanische Auslenkung und/oder ein Schalldruck ist, wobei die charakteristische Größe gegebenenfalls Amplituden- und/oder Phaseninformationen beinhaltet.

3. Elektrodenleitung (530, 531) nach einem der vorhergehenden Ansprüche, **dadurch gekennzeichnet, dass** zur Bestimmung der mindestens einen individuellen Eigenschaft mindestens ein lokales Extremum (115) und/oder mindestens ein Wendepunkt (116) und/oder die erste und/oder eine höhere Ableitung der mindestens einen gemessenen Kennlinie (110) bestimmbar ist, wobei die mindestens eine gemessene Kennlinie (110) vorzugsweise eine Zeitabhängigkeit, eine Frequenzabhängigkeit (251), eine Spannungsabhängigkeit (410) und/oder eine Stromabhängigkeit der charakteristischen Größe beinhaltet.

4. Elektrodenleitung (530, 531) nach Anspruch 3, **dadurch gekennzeichnet, dass** der ID-Schaltkreis (550, 551, 1550, 2550)
mindestens einen Resonator, vorzugsweise zwei oder mehrere parallel geschaltete Resonatoren, und/oder
mindestens einen nichtlinearen Spannungsteiler, vorzugsweise zwei oder mehrere kaskadisch geschaltete nichtlineare Spannungsteiler, jeweils mit mindestens einer Nichtlinearität, und/oder
mindestens eine Verzögerungseinheit (850, 851), vorzugsweise mehrere parallel und/oder in Reihe angeordnete Verzögerungseinheiten (850, 851), aufweist.

5. Elektrodenleitung (530, 531) nach Anspruch 4, **dadurch gekennzeichnet, dass** der Resonator als LC-Schwingkreis (220), gegebenenfalls mit einem Dämpfungsglied, vorzugsweise in Form eines elektrischen Widerstands (230),
oder als Piezoelement und/oder SAW-Resonator ausgebildet ist.

6. Elektrodenleitung (530, 531) nach einem der Ansprüche 4 bis 5, **dadurch gekennzeichnet, dass** die Verzögerungseinheit (850, 851) einen bidirektionalen Signal-Welle-Wandler (800, 801), eine Verzögerungsstrecke (810, 811) und einen Reflektor (820, 821) aufweist, wobei ein Signal durch den Signal-Welle-Wandler (800, 801) in eine Welle umwandelbar ist, welche sich über die Verzögerungsstrecke (810, 811) bis zum mit dieser verbundenen Reflektor (820, 821) ausbreitet und zum Signal-Welle-Wandler (800, 801) zurück reflektierbar ist.

7. Elektrodenleitung (530, 531) nach einem der vorhergehenden Ansprüche, **dadurch gekennzeichnet, dass** die Elektrodenleitung (530, 531) mindestens einen Energiespeicher, beispielsweise einen Kondensator und/oder eine Sekundärzelle, aufweist, der mit dem ID-Schaltkreis (550, 551, 1550, 2550) verbunden ist.

8. Verfahren zur Identifikation einer Elektrodenleitung (530, 531) nach einem der Ansprüche 1 bis 7, umfassend die folgenden Schritte:
- Anregen des ID-Schaltkreises (550, 551, 1550, 2550) über die Koppeleinrichtung (525, 560) der Elektrodenleitung (530, 531),
- Messen der mindestens einen Kennlinie (110) der charakteristischen Größe des ID-Schaltkreises (550, 551, 1550, 2550),
- Bestimmen der mindestens einen individuellen Eigenschaft der Elektrodenleitung (530, 531), basierend auf der mindestens einen gemessenen Kennlinie.

9. Verfahren nach Anspruch 8, **dadurch gekennzeichnet, dass** das Bestimmen der mindestens einen individuellen Eigenschaft folgende Schritte umfasst:
- Bestimmen mindestens eines lokalen Extremums (115) und/oder mindestens eines Wendepunkts (116) und/oder der ersten und/oder einer höheren Ableitung der mindestens einen gemessenen Kennlinie (110),
- Ermitteln eines Codes der mindestens einen individuellen Eigenschaft auf Basis des bestimmten mindestens einen Extremums (115) und/oder mindestens einen Wendepunkts (116).

10. Verfahren nach Anspruch 8, **dadurch gekennzeichnet, dass** das Bestimmen der mindestens einen individuellen Eigenschaft folgende Schritte umfasst:
- Vergleichen der mindestens einen gemessenen Kennlinie (110) der charakteristischen Größe mit mindestens einer gespeicherten Kennlinie,
- Ermitteln eines Codes der mindestens einen individuellen Eigenschaft auf der Basis des Ergebnisses des Vergleichs.

11. Implantat (500) mit einer Buchse zur Verbindung mit dem Stecker (511, 512) einer Elektrodenleitung (530, 531) nach einem der Ansprüche 1 bis 7, mit einer an oder in der Buchse angeordneten Koppeleinrichtung (526, 561) sowie einer Identifizierungseinrichtung (501),
wobei die Identifizierungseinrichtung (501, 1501) derart ausgestaltet ist, dass mittels der Koppeleinrichtung (526, 561) des Implantats (500) über die Koppeleinrichtung (525, 560) der Elektrodenleitung (530, 531) der ID-Schaltkreis (550, 551, 1550, 2550) der Elektrodenleitung (530, 531) anregbar ist, dass während oder nach der Anregung die charakteristische Größe des ID-Schaltkreises (550, 551, 1550, 2550) über die Koppeleinrichtung (525, 560) der Elektrodenleitung (530, 531) und des Implantats (500) und/oder über die Auskoppeleinrichtung der Elektrodenleitung (530, 531) messbar ist und dass auf der Basis der mindestens einen gemessenen Kennlinie (110) der charakteristischen Größe die mindestens eine individuelle Eigenschaft der Elektrodenleitung (530, 531) bestimmbar ist.

12. Implantat (500) nach Anspruch 11, **dadurch gekennzeichnet, dass** die Identifizierungseinrichtung (501, 1501) einen Code der mindestens einen 2- individuellen Eigenschaft bestimmen kann.

13. Implantat (500) nach Anspruch 12, **dadurch gekennzeichnet, dass** die Identifizierungseinrichtung (501, 1501) mindestens ein lokales Extremum (115) und/oder mindestens einen Wendepunkt (116) und/oder die erste und/oder eine höhere Ableitung der mindestens einen gemessenen Kennlinie (110) bestimmen und den Code der mindestens einen individuellen Eigenschaft auf Basis des bestimmten mindestens einen Extremums (115) und/oder mindestens einen Wendepunkts (116) ermitteln kann.

14. Implantat (500) nach Anspruch 12, **dadurch gekennzeichnet, dass** die Identifizierungseinrichtung (501, 1501) die mindestens eine gemessene Kennlinie (110) der charakteristischen Größe mit mindestens einer gespeicherten Kennlinie (110) vergleichen und den Code der mindestens einen individuellen Eigenschaft auf der Basis des Ergebnisses des Vergleichs ermitteln kann.

## Claims

1. Electrode lead (530, 531) for connection to an implant (500) by means of a plug (511, 512),
**characterised by** an analogue electric ID circuit (550, 551), which is arranged in the electrode lead (530, 531) on the plug side and is connected to a coupling device (525, 560) of the electrode lead (530, 531), which coupling device is arranged on the plug side,
the ID circuit (550, 551, 1550, 2550) being excitable via the coupling device (525, 560) and being designed in such a way that, during or after the excitation, a characteristic variable of the ID circuit (550, 551, 1550, 2550) is measurable via the coupling device (525, 560) and/or via an output device of the electrode lead (530, 531), which output device is connected to the ID circuit (550, 551, 1550, 2550) and is arranged on the plug side, and in such a way that at least one individual property of the electrode lead (530, 531) is determinable on the basis of at least one measured characteristic curve (110) of the characteristic variable.

2. Electrode lead (530, 531) according to claim 1, **characterised in that** the characteristic variable is a voltage, a current (420), an impedance (254), a frequency, a mechanical deflection and/or a sound pressure, the characteristic variable possibly including amplitude and/or phase information.

3. Electrode lead (530, 531) according to one of the preceding claims, **characterised in that**, in order to determine the at least one individual property, at least one local extremum (115) and/or at least one inflection point (116) and/or the first and/or a higher derivative of the at least one characteristic curve (110) is determinable, the at least one measured characteristic curve (110) preferably including a time dependency, a frequency dependency (251), a voltage dependency (410) and/or a current dependency of the characteristic variable.

4. Electrode lead (530, 531) according to claim 3, **characterised in that** the ID circuit (550, 551, 1550, 2550) comprises
at least one resonator, preferably two or more resonators connected in parallel, and/or at least one non-linear voltage divider, preferably two or more non-linear voltage dividers connected in cascade, each having at least one non-linearity, and/or
at least one delay unit (850, 851), preferably a plurality of delay units (850, 851) arranged in parallel and/or in series.

5. Electrode lead (530, 531) according to claim 4, **characterised in that** the resonator is designed as an LC resonant circuit (220), optionally with a damping member, preferably in the form of an electric resistor (230), or is designed as a piezoelement and/or SAW resonator.

6. Electrode lead (530, 531) according to one of claims 4 to 5, **characterised in that** the delay unit (850, 851) comprises a bidirectional signal-wave converter (800, 801), a delay section (810, 811), and a reflector (820, 821), a signal being convertible by the signal-wave converter (800, 801) into a wave which propagates over the delay path (810, 811) as far as the reflector (820, 821) connected thereto and is reflectable back to the signal-wave converter (800, 801).

7. Electrode lead (530, 531) according to one of the preceding claims, **characterised in that** the electrode lead (530, 531) comprises at least one energy store, for example a capacitor and/or a secondary cell, which is connected to the ID circuit (550, 551, 1550, 2550).

8. Method for identifying an electrode lead (530, 531) according to one of claims 1 to 7, comprising the following steps:
- exciting the ID circuit (550, 551, 1550, 2550) via the coupling device (525, 560) of the electrode lead (530, 531),
- measuring the at least one characteristic curve (110) of the characteristic variable of the ID circuit (550, 551, 1550, 2550),
- determining the at least one individual property of the electrode lead (530, 531) on the basis of the at least one measured characteristic curve.

9. Method according to claim 8, **characterised in that** the determination of the at least one individual property comprises the following steps:
- determining at least one local extremum (115) and/or at least one inflection point (116) and/or the first and/or a higher derivative of the at least one measured characteristic curve (110),
- ascertaining a code of the at least one individual property on the basis of the determined at least one extremum (115) and/or at least one inflection point (116).

10. Method according to claim 8, **characterised in that** the determination of the at least one individual property comprises the following steps:
- comparing the at least one measured characteristic curve (110) of the characteristic variable with at least one stored characteristic curve,
- ascertaining a code of the at least one individual property on the basis of the result of the comparison.

11. Implant (500) with a socket for connection to the plug (511, 512) of an electrode lead (530, 531) according to one of claims 1 to 7, with a coupling device (526, 561) arranged on or in the socket and also with an identification device (501),
the identification device (501, 1501) being designed in such a way that, by means of the coupling device (526, 561) of the implant (500), the ID circuit (550, 551, 1550, 2550) of the electrode lead (530, 531) is excitable via the coupling device (525, 560) of the electrode lead (530, 531), during or after the excitation the characteristic variable of the ID circuit (550, 551, 1550, 2550) is measurable via the coupling device (525, 560) of the electrode lead (530, 531) and of the implant (500) and/or via the output device of the electrode lead (530, 531), and the at least one individual property of the electrode lead (530, 531) is determinable on the basis of the at least one measured characteristic curve (110) of the characteristic variable.

12. Implant (500) according to claim 11, **characterised in that** the identification device (501, 1501) is able to determine a code of the at least one individual property.

13. Implant (500) according to claim 12, **characterised in that** the identification device (501, 1501) is able to determine at least one local extremum (115) and/or at least one inflection point (116) and/or the first and/or a higher derivative of the at least one measured characteristic curve and is able to ascertain the code of the at least one individual property on the basis of the determined at least one extremum (115) and/or at least one inflection point (116).

14. Implant (500) according to claim 12, **characterised in that** the identification device (501, 1501) is able to compare the at least one measured characteristic curve (110) of the characteristic variable with at least one stored characteristic curve (110) and is able to ascertain the code of the at least one individual property on the basis of the result of the comparison.

## Revendications

1. Ligne d'électrode (530, 531) permettant la liaison avec un implant (500) au moyen d'un connecteur (511, 512),
**caractérisée par** un circuit d'identification (550, 551) électrique analogique qui est disposé dans la ligne d'électrode (530, 531) du côté connecteur et est relié avec un dispositif de couplage (525, 560) de la ligne d'électrode (530, 531) disposé du côté connecteur,
où le circuit d'identification (550, 551, 1550, 2550) peut être excité par le biais du dispositif de couplage (525, 560) et est conçu de sorte que, pendant ou après l'excitation, une grandeur caractéristique du circuit d'identification (550, 551, 1550, 2550) peut être mesurée par le biais du dispositif de couplage (525, 560) et/ou par le biais d'un dispositif de découplage de la ligne d'électrode (530, 531) disposé du côté connecteur, relié avec le circuit d'identification (550, 551, 1550, 2550) et qu'au moins une propriété individuelle de la ligne d'électrode (530, 531) peut être déterminée sur la base d'au moins une courbe caractéristique (110) mesurée de la grandeur caractéristique.

2. Ligne d'électrode (530, 531) selon la revendication 1, **caractérisée en ce que** la grandeur caractéristique est une tension, un courant (420), une impédance (254), une fréquence, une déflexion mécanique et/ou une pression acoustique, où la grandeur caractéristique contient éventuellement des informations sur des amplitudes et/ou des phases.

3. Ligne d'électrode (530, 531) selon l'une des revendications précédentes, **caractérisée en ce que**, pour la détermination de l'au moins une propriété individuelle, au moins une valeur extrême (115) locale, et/ou au moins un point d'inflexion (116), et/ou la dérivée première, et/ou une dérivée d'ordre plus élevé de l'au moins une courbe caractéristique (110) mesurée, puissent être déterminés,
où l'au moins une courbe caractéristique (110) mesurée contient de préférence une fonction du temps, une fonction de la fréquence (251), une fonction de la tension (410) et/ou une fonction du courant de la grandeur caractéristique.

4. Ligne d'électrode (530, 531) selon la revendication 3, **caractérisée en ce que** le circuit d'identification (550, 551, 550, 2550) présente
au moins un résonateur, de préférence deux ou plusieurs résonateurs branchés en parallèle, et/ou
au moins un diviseur de tension non linéaire, de préférence deux ou plusieurs diviseurs de tension non linéaires branchés en cascade, avec respectivement une non linéarité, et/ou
au moins une unité de retard (850, 851), de préférence, plusieurs unités de retard (850, 851) disposées en parallèle et/ou en série.

5. Ligne d'électrode (530, 531) selon la revendication 4, **caractérisée en ce que** le résonateur est conçu sous forme d'un circuit oscillant LC (220), éventuellement avec un organe d'amortissement, de préférence, sous la forme d'une résistance (230) électrique,
ou sous la forme d'un élément piézo et/ou d'un résonateur SAW.

6. Ligne d'électrode (530, 531) selon l'une des revendications 4 à 5, **caractérisée en ce que** l'unité de retard (850, 851) présente un convertisseur onde-signal (800, 801) bidirectionnel, une ligne de retard (810, 811) et un réflecteur (820, 821), où un signal peut être converti en une onde par le convertisseur onde-signal (800, 801), laquelle s'étend sur la ligne de retard (810, 811) jusqu'au réflecteur (820, 821) y étant relié et est de nouveau réfléchie vers le convertisseur onde-signal (800, 801).

7. Ligne d'électrode (530, 531) selon l'une des revendications précédentes, **caractérisée en ce que** la ligne d'électrode (530, 531) présente au moins un accumulateur d'énergie, par exemple, un condensateur, et/ou une cellule secondaire, qui est relié avec le circuit d'identification (550, 551, 1550, 2550).

8. Procédé d'identification d'une ligne l'électrode (530, 531) selon l'une des revendications 1 à 7, comprenant les étapes suivantes :
- l'excitation du circuit d'identification (550, 551, 1550, 2550) par le biais du dispositif de couplage (525, 560) de la ligne d'électrode (530, 531),
- la mesure de l'au moins une courbe caractéristique (110) de la grandeur caractéristique du circuit d'identification (550, 551, 1550, 2550),
- la détermination de l'au moins une propriété individuelle de la ligne d'électrode (530, 531) en se basant sur l'au moins une courbe caractéristique mesurée.

9. Procédé selon la revendication 8, **caractérisé en ce que** la détermination de l'au moins une propriété individuelle comprend les étapes suivantes :
- la détermination d'au moins une valeur extrême (115) locale, et/ou d'au moins un point d'inflexion (116), et/ou de la dérivée première, et/ou d'une dérivée d'ordre plus élevé de l'au moins une courbe caractéristique (110) mesurée,
- l'évaluation d'un code de l'au moins une propriété individuelle sur la base de l'au moins une valeur extrême (115) et/ou de l'au moins un point d'inflexion (116) déterminés.

10. Procédé selon la revendication 8, **caractérisé en ce que** la détermination de l'au moins une propriété individuelle comprend les étapes suivantes :
- la comparaison de l'au moins une courbe caractéristique (110) mesurée de la grandeur caractéristique avec au moins une courbe caractéristique enregistrée,
- l'évaluation d'un code de l'au moins une propriété individuelle sur la base du résultat de la comparaison.

11. Implant (500) avec une douille pour la connexion avec le connecteur (511, 512) d'une ligne d'électrode (530, 531) selon l'une des revendications 1 à 7, avec un dispositif de couplage (526, 561) disposé au niveau de la douille ou dans la douille, ainsi d'un dispositif d'identification (501),
dans lequel le dispositif d'identification (501, 1501) est conçu de manière à ce que, au moyen du dispositif de couplage (526, 561) de l'implant (500), le circuit d'identification (550, 551, 1550, 2550) de la ligne d'électrode (530, 531) puisse être excité par le biais du dispositif de couplage (525, 560) de la ligne d'électrode (530, 531) de sorte que, pendant ou après l'excitation, la grandeur caractéristique du circuit d'identification (550, 551, 1550, 2550) est mesurable par le biais du dispositif de couplage (525, 560) de la ligne d'électrode (530, 531) et de l'implant (500), et/ou par le biais du dispositif de découplage de la ligne d'électrode (530, 531), et que, sur la base de l'au moins une courbe caractéristique (110) mesurée de la grandeur caractéristique, l'au moins une propriété individuelle de la ligne d'électrode (530, 531) puisse être déterminée.

12. Implant (500) selon la revendication 11, **caractérisé en ce que** le dispositif d'identification (501, 1501) peut déterminer un code de l'au moins une propriété individuelle.

13. Implant (500) selon la revendication 12, **caractérisé en ce que** le dispositif d'identification (501, 1501) peut déterminer au moins une valeur extrême (115) locale, et/ou au moins un point d'inflexion (116), et/ou la dérivée première, et/ou une dérivée d'ordre plus élevé de l'au moins une courbe caractéristique (110) mesurée, et puisse évaluer le code de l'au moins une propriété individuelle sur la base de l'au moins une valeur extrême (115) et/ou de l'au moins un point d'inflexion (116) déterminés.

14. Implant (500) selon la revendication 12, **caractérisé en ce que** le dispositif d'identification (501, 1501) est capable de comparer l'au moins une courbe caractéristique (110) mesurée de la grandeur caractéristique avec au moins une courbe caractéristique (110) enregistrée, et peut évaluer le code de l'au moins une propriété individuelle sur la base du résultat de la comparaison.
